(19) Europäisches Patentamt
European Patent Office
Office européen des brevets

(11) EP 2 260 902 A1

(12) **EUROPEAN PATENT APPLICATION**

(43) Date of publication:
**15.12.2010 Bulletin 2010/50**

(51) Int Cl.:
***A61N 5/10*** *(2006.01)*

(21) Application number: **09162436.1**

(22) Date of filing: **10.06.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**

(71) Applicant: **Deutsches Krebsforschungszentrum Stiftung des öffentlichen Rechts**
**69120 Heidelberg (DE)**

(72) Inventors:
- **Ziegenhein, Peter**
  **69120 Heidelberg (DE)**
- **Oelfke, Uwe**
  **69120 Heidelberg (DE)**

(74) Representative: **Lucke, Andreas**
**Forrester & Boehmert**
**Pettenkoferstrasse 20-22**
**80336 München (DE)**

(54) **Radiation treatment planning system and computer program product**

(57) The present invention relates to a radiation treatment planning system and a corresponding computer program product. The system comprises means for graphically displaying an image representing a target area 10 to be treated with a set of therapeutic radiation beams and an adjacent structure comprising healthy tissue 14 and/or organs at risk 12, and for displaying corresponding dose values according to a preliminary treatment plan. The system further comprises means for allowing a user to interactively input a local dose variation, local dose variation means for revising the preliminary treatment plan such as to account for the local dose variation inputted by the user and dose recovery means comprising means for revising the treatment plan again such as to at least partially compensate for a change of dose in a predetermined recovery area caused by said dose variation.

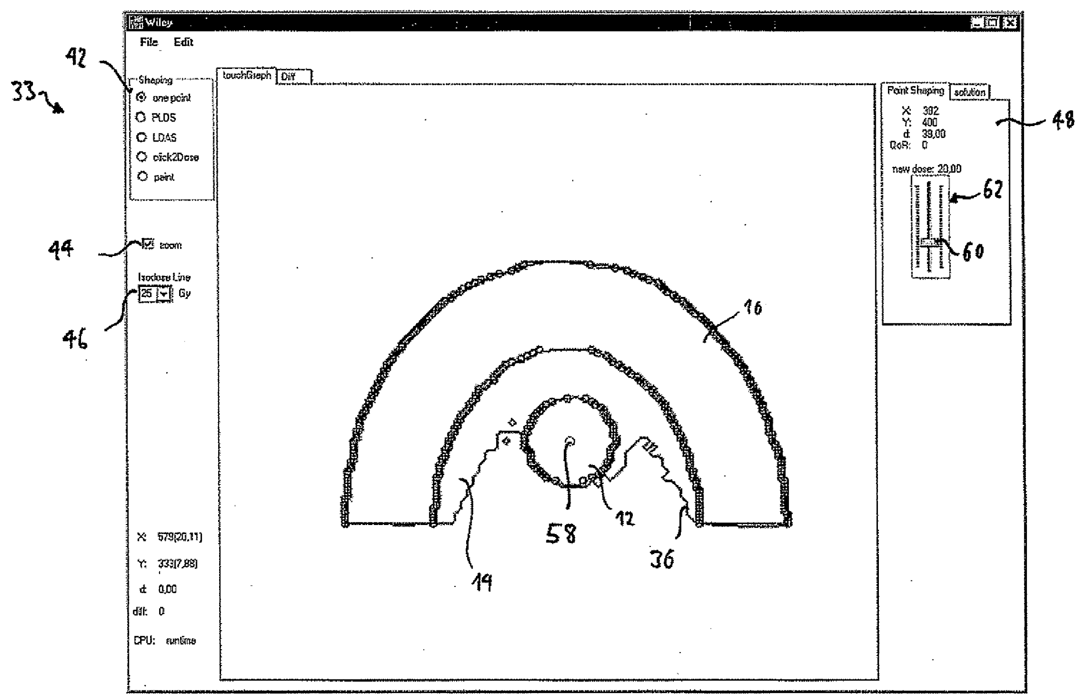


Fig. 11



EP 2 260 902 A1

## Description

### BACKGROUND OF THE INVENTION

### Field of the Invention

The present invention relates generally to radiation therapy. More specifically, the present invention relates to a system and a computer program product for radiation treatment planning.

### Description of the Related Art

Radiation therapy can be effective in treating cancers, tumors, lesions or other targets. Many tumors can be eradicated completely if a sufficient radiation dose is delivered to the tumor body such as to destroy the tumor cells. The maximum dose which can be applied to a tumor is determined by the tolerance dose of the surrounding healthy tissue. With the development of computer tomography (CT) and magnet resonance tomography (MRT), sophisticated insight into the body of the patient has been given. Supported by the increasing availability of high-performance computers, a new generation of treatment planning systems for a conformal 3D-therapy technique has been developed which allows to increase the ratio between the tumor dose and the dose applied to surrounding healthy tissue.

A very successful technique in this regard is the so called intensity modulated radiation therapy (IMRT). The basic idea of IMRT is to modulate the cross-sectional beam intensity profile in a suitable way such as to obtain a higher spatial conformity of the resulting dose distribution with the planned target volumes obtained previously by CT or MRT images of the patient. To explain the basic concept of IMRT in more detail, reference is made to Fig. 1. On the left side of Fig. 1, a tumor is shown which is irradiated with three radiation beams each having a constant intensity profile. As can be seen from the left side of Fig. 1, by irradiating with constant profile beams, the radiation dose cannot be precisely confined to the volume of the tumor, but may also affect an organ at risk (OAR) located in the vicinity of the tumor.

In comparison, in IMRT the cross-section intensity of the beams are modulated such as to deliver a high dose to the tumor but as little dose as possible to the surrounding healthy tissue or organs at risk, as is illustrated in the right side of Fig. 1.

In order to parameterize the cross-sectioned intensity profile of the beam, the beam is usually discretized in a number of "beamlets" or "bixels", and a certain intensity or weight is associated with each bixel. In other words, a beam profile can completely be parameterized by a set of bixel weights or a bixel-weight-array called "bixel-array" for short, and the bixel-arrays for all beam directions used in the treatment constitute a treatment plan. Once the bixel-arrays are know, the corresponding beam intensities can be generated in a radiation therapy apparatus using multi-leaf collimators or other beam shaping techniques.

Once a set of bixel-weight-arrays is known, it is also easy and straightforward to calculate the corresponding dose distribution in the patient. However, unfortunately in practice the problem to be solved is the other way around: Based on detailed knowledge of the patient geometry from CT or MRT images, the radiooncologist prescribes a certain dose distribution within the target area and certain dose constraints in the organs at risk, and the problem is to find the corresponding bixel weights to achieve this. This problem is called "inverse planning" for obvious reasons. The goal is to find a set of bixel weights resulting in a treatment plan which is as close as possible to the prescribed dose distribution. To perform this inverse planning, optimization modules have been developed which find appropriate bixel weights in an iterative search using a suitable cost function, as is for example described in Nill, S. "Development and application of a multi-modality inverse planning system." Ph.D. thesis, University of Heidelberg. URL http://www.ub.uni-heidelberg.de/archiv/1802. Such iterative search can take between several minutes and several hours due to the complexity of the problem.

While such prior art optimization modules have been extremely useful in devising treatment plans, there are still a number of problems remaining.

First of all, due to the global optimization scheme, the quality of a given treatment plan will be judged with reference to a number of global quality criteria. However, the global optimization often cannot prevent adverse local effects in the treatment plan. An example for such local effects are hot spots, i.e. strictly localized dose maxima, which due to their strict locality have only little influence on the cost functions used in the optimization scheme but are of course clinically prohibitive.

Another problem involved with prior art global optimization modules is that due to extended computation time, the treatment plan has to be calculated well in advance of the actual treatment and is therefore often based on medical images that have been taken several days or even weeks prior to the treatment. If the patient geometry changes between taking the planning images and the actual therapy, for example due to tumor growth, the treatment plan may no longer be quite suitable. Also, since the treatment plan is based on a global optimization scheme, there is no room for local corrections.

Finally, with prior art optimization methods, the radiotherapist has very little influence on the optimization process. The mathematical search for the bixel weight runs largely automatically and is only governed by the cost function, without interaction by the therapist. Accordingly, the prior art optimization modules lack flexibility and interaction with the radiotherapist.

In US 6,661,870, a method of compensating for unexpected changes in the size, shape and position of a patient in the delivery of radiation therapy is described. According to this prior art method, a first image of a tumor region in a patient to be treated is obtained, and a treatment plan is created based on this first image. When the actual treatment is to be performed, a second image of the tumor region will be obtained, and the treatment is modified on-line based on changes in the tumor region in the patient as represented in the second image. It is suggested to manually adjust the amount of radiation for selected voxels in the voxel-grid of the treatment plan without re-optimizing the full treatment plan. However, such manual adjustment is again difficult to perform, because any adjustment of a bixel to correct a dose locally will also have an effect on the dose distribution at other sites. In fact, due to the complexity and the inherent synergistic effect of IMRT, a suitable manual revision of the treatment plan is rather difficult to perform.

Accordingly, it is an object of the invention to provide a radiation treatment planning system and computer program product which help to overcome the above mentioned problems.

This object is achieved by a radiation treatment planning system comprising:

- means for graphically displaying an image representing a target area to be treated with a set of therapeutic radiation beams, an adjacent structure comprising healthy tissue and/or organs at risk and for displaying corresponding dose values according to an initial or a preliminary treatment plan,
- means for allowing a user to interactively input a local dose variation,
- local dose variation means for revising the initial or preliminary treatment plan such as to account for the local dose variation inputted by the user, and
- dose recovery means comprising means for revising the treatment plan again such as to at least partially compensate for a change of dose in a predetermined recovery area caused by said dose variation.

According to the invention, the user can visually inspect the dose distribution in an image representing the target area and the surrounding tissue corresponding to a given treatment plan and can interactively input a local dose variation wherever he or she sees room for improvement. Herein, the "given treatment plan" can be an initial treatment plan, which could be any starting point when the treatment plan is designed from scratch or; a pre-optimized plan which possibly has been obtained by other means. In the following, an explicit distinction between an initial or preliminary treatment plan is no longer made, where the term "preliminary treatment plan" is understood to refer to any starting point for a local dose shaping, as will become more apparent from the description of the specific embodiment below.

Upon the input by the user, the preliminary treatment plan can be revised by local dose variation means such as to account for the inputted local dose variation. For example, the local dose variation means can compute suitable adjustments of bixel weights such that the local dose will change as prescribed by user input. However, every change of beam intensity will not only effect the dose at the site of local dose variation, but also in remote sites, where no change in the dose is wanted, because the dose may already be suitable. Due to the high synergistic effect of methods like IMRT, a local dose variation to the better will generally lead to a change of dose at a remote site to the worse. According to the system of the invention, dose recovery means are provided which comprise means for revising the treatment plan again such as to at least partially compensate for a change of dose in a predetermined recovery area caused by dose variation. Herein, the "predetermined recovery area" is an area where no change of dose due to the local dose variation is wanted.

Due to the interplay of the local dose variation and dose recovery, the therapist is in a position to interactively locally change the dose without overthrowing the whole treatment plan. The combination of local dose variation and a consecutive dose recovery is called "local dose shaping" herein.

Accordingly, due to the system of the invention, the radiotherapist can interact with the treatment planning system such as to step by step improve the treatment plan. This is for example advantageous in cases where a fairly good treatment plan for example obtained by a prior art optimization method is available but only some local hot spots or cold spots need to be corrected for, or where some local changes are necessary due to a change of patient geometry, for example due to tumor growth. However, the concept of local dose shaping even allows to devise the treatment plan interactively completely from scratch.

While it is believed that due to the complexity and synergistic effects between the numerous bixels constituting the treatment beams reasonable treatment plans cannot be derived manually, the local dose shaping scheme of the invention does in fact allow just this. Of course, the interrelations of the various bixels are also present in the framework of the local dose shaping, but they are accounted for and made "invisible" to the user by dose recovery means, as will become more apparent with reference to the exemplary embodiments below.

In one embodiment, the treatment to be planned by the system comprises IMRT employing therapeutic beams

radiated from different directions and each having a modulated cross-sectional beam intensity profile, wherein the treatment plan comprises data representing a set of cross-sectional beam intensity profiles, one for each radiation direction, and in particular, a set of bixel-arrays, each bixel-array representing a corresponding beam intensity profile in a discretized manner.

However, the invention is by no means limited to IMRT. For example, the invention is also applicable to a so-called "open-field radiation therapy", in which for each beam the shape and size of the radiation field, i.e. its boundary can be modulated and a uniform beam intensity can be chosen, but where the intensity within the radiation field itself remains uniform. Note that conceptionally this may be regarded as a variant of IMRT, where the intensity modulation for each pixel corresponds to zero or 100% of the beam's intensity. Accordingly, when in the following description reference is made to IMRT, the respective disclosure is also meant to apply for open field radiation therapy, even though this will not explicitly mentioned. For completeness, a further important radiation therapy modality is the so-called "rotation-therapy", in which open fields from as much as for example 36 different directions are applied. Again, this method is conceptionally closely related to the other two, but is referred to by a different name in the field. It is emphasized that the present invention is intended to be employed for each of these modalities, even though in the following description, specific reference to IMRT will be made.

In a preferred embodiment, the means for allowing a user to interactively input a local dose variation comprise means for allowing a user to manually select one or more individual points within the image and to change the corresponding dose value, or to manually shift an isodose curve displayed in said image using an input device. This way, the user can very intuitively and easily interact with the system such as to carry out a local dose shaping. In a further preferred embodiment, the user may input a local dose variation by manually shifting a graph representing a dose-value-histogram to a desired value. In this embodiment, further means are provided to automatically determine single bixels for a local dose variation which in combination will lead to the modified DVHRs inputted by the user. In other words, there are numerous ways for a user to input a local dose variation directly or indirectly, which are all encompassed by the present invention.

Preferably, the local dose variation means are configured to perform the steps of

- selecting, for every beam, a subset of bixels, and
- adjusting the intensities of the selected bixels by a predetermined mathematical operation ensuring an overall change of the local dose related to the inputted local dose variation.

Herein, the subset of bixels selected can be formed by the single bixel of each beam contributing the most to the local dose, a predetermined number of bixels contributing the most to the local dose or the subset of bixels having relative contributions to the local dose which exceed a predetermined threshold.

By suitable choice of the predetermined threshold, the burden of the dose variation can be distributed on a suitable number of bixels. The lower the threshold, the more bixels will be involved, allowing for smaller changes of the respective weights. However, at the same time more unwanted dose deviation may occur in uninvolved voxels within a wide location around the local dose variation site.

In a preferred embodiment, the predetermined recovery area comprises a set of predetermined voxels on which the recovery process is to be carried out, and the dose recovery means are configured to

a) select one of the voxels according to a predetermined selection strategy,
b) revise the treatment plan such as to at least partially recover the dose at the selected voxel, and
c) compute the revised dose distribution according to the revised treatment plan,

wherein the steps a) to c) are repeated until a predetermined stop criterion is met.

According to this embodiment, the recovery is obtained iteratively, where each iteration step starts out with selecting one of the voxels on which the recovery process is to be carried out. Herein, the predetermined selection strategy may be based on the absolute value of the dose difference to be compensated, meaning that the recovery starts at those voxels that have been disturbed the most by the previous local dose variation. Due to the iteration scheme, the recovery will be repeated for a number of cycles and in general every cycle will start at a different site. This way, the dose distribution in the predetermined recovery area will converge to the dose distribution prior to the local dose variation. Note that in addition to the absolute value of the dose difference to be compensated, the selection strategy can also be based on the absolute value of the distance of the voxel from the location of local dose variation.

As regards the stop criterion, it may be based on a certain quality of the recovery reached and/or on a number of iterations of steps a) to c). In an actual embodiment, it was found that 50 to 100 dose recovery iterations were necessary to find a suitable convergence and that the calculation time needed was about 1 second, allowing for a truly interactive and on the fly local dose shaping.

In a preferred embodiment, the system employs a dose-array comprising a first set of voxels resembling the

treatment volume with a first resolution, wherein a dose value is assigned to each voxel of said first set of voxels, and the system further employs a dose-grid comprising a second set of voxels resembling the treatment volume with a second resolution lower than said first resolution, wherein a dose value is assigned to each voxel of said second set of voxels, wherein the dose recovery means employ the dose-grid for the dose recovery.

By using the dose-grid with a lesser resolution and performing the dose recovery thereon, the amount of data needed when performing the computations of the recovery steps will be small enough to be included in the higher memory, thus avoiding a von-Neumann bottleneck that would otherwise slow down the computation dramatically. It has been found that for the purpose of the recovery calculations, a fairly sparse resolution will be sufficient.

The use of memory can be further optimized if the dose-grid comprises at least two sub-grids having different resolutions, the different resolutions being associated with at least two different tissue classes, said different tissue classes being selected from the group consisting of target tissue, healthy tissue and tissue of an organ at risk. As will be explained below with reference to a specific embodiment, for the purpose of computing the dose recovery, a smallest dose-grid resolution is acceptable for healthy tissue and a highest resolution is preferable for an organ at risk.

Further, in step b) mentioned above, for every beam one or more bixels contributing most to the dose of the selected voxel is or are preferably selected among the bixels that had not been involved in the local dose variation, and the intensities of each selected bixel are adjusted according to a predetermined mathematical operation ensuring a change of the dose at the selected voxel such as to at least approximately recover the original dose.

Herein, assuming that the selected voxel is located in one of the tissue classes target, healthy tissue and OAR, the mathematical operation preferably also accounts for the impact each selected bixel has on the tissue of the main two tissue classes. This can for example be achieved by introducing geometrical factors accounting for the distance a certain bixel transverses in a tissue of the remaining two tissue classes. This way, the bixels having the most suitable path or direction will be made to contribute the most to the dose recovery, thus further improving the quality thereof.

In a preferred embodiment, the system is configured to perform, in response to an inputted dose variation, a sequence of alternating local dose variation and dose recovery steps, wherein in each of the local dose variation steps, the local dose variation is performed for a predetermined fraction of the inputted local dose variation value only. This embodiment is an "adiabatic" approach, where the local change of dose prescribed by the user will be split up in a number of steps of smaller local dose variation with dose recovery steps inbetween. It has been confirmed in experiment that this adiabatic approach allows for a very smooth and stable convergence of the local dose shaping.

Disclosed herein is also a method for planning a radiation treatment, comprising the steps of:

- graphically displaying an image representing a target area to be treated with a set of therapeutic radiation beams, an adjacent structure comprising healthy tissue and/or organs at risk and displaying a dose distribution according to a preliminary treatment plan,
- receiving an input of a local dose variation,
- revising the preliminary treatment plan such as to account for the local dose variation received, and
- revising the treatment plan again such as to at least partially compensate for a change of dose in a predetermined recovery area caused by said dose variation

## BRIEF DESCRIPTION OF THE FIGURES

| | |
|---|---|
| Fig. 1 | is a schematic diagram for explaining the intensity modulated radiation therapy concept. |
| Fig. 2a, b | show the relative weight and the absolute contribution of bixels, if all bixels are considered in the variation process. |
| Fig. 3a, b | show the same diagrams as Fig. 2a, b but for a case where only a single bixel is changed for accounting for the local dose variation. |
| Fig. 4 | is a symbolic representation of the data structure used in an embodiment of the invention. |
| Fig. 5 | shows a phantom setup and an overlying dose-grid structure. |
| Fig. 6 | shows a work flow of a local dose shaping step. |
| Fig. 7 | is a schematic diagram showing the bixels involved in the dose recovery of a voxel. |
| Fig. 8 to 17 | are screenshots of a GUI provided by a system according to an embodiment of the invention under operation. |
| Fig. 18 | is a schematic diagram showing the inputting of a local dose variation by shift- ing isodose lines. |

## DETAILED DESCRIPTION OF A PREFERRED EMBODIMENT

For the purposes of promoting an understanding of the principles of the invention, reference will now be made

to the preferred embodiment illustrated in the drawings and specific language will be used to describe the same. It will nevertheless be understood that no limitation of the scope of the invention is thereby intended, such alterations and further modifications in the illustrated system and such further applications of the principles of the invention as illustrated therein being contemplated as would normally occur now or in the future to one skilled in the art to which the invention relates.

In the following, a preferred embodiment of a radiation treatment planning system according to the present invention will be described. To begin with, however, the underlying concept of local dose shaping, the preferred data structure used and a work flow of local dose shaping employed in the planning system will be explained in detail.

## The Concept of Local Dose Shaping

The concept of local dose shaping is a new approach for inverse radiation treatment planning which allows to break up the automatic optimization algorithms used in prior art and allows for more involvement and interaction of a radiation therapist in the planning. The local dose shaping according to the invention comprises two basic steps, a *local dose variation step* and *a dose recovery step*. The local dose variation step implements a local planning goal which is inputted interactively by a user. In the easiest case, the user may select one or more individual points or voxels within a graphical image representing the treatment volume and input a new dose value differing from the present one. Upon this input, the radiation treatment planning system will revise the treatment plan such as to account for the local dose variation inputted by the user. In practice this means that the intensities or weights of certain bixels of the radiation beams will be modified such as to yield the prescribed local dose value at the selected site. However, the change of the bixel weight will also affect the dose at places remote from the selected site. According to the invention, the radiation treatment is configured to conduct a dose recovery step that restores the original dose, i.e. the dose prior to the local dose variation in a predetermined recovery area, where the dose was not intended to be changed.

## Modification of local dose

The starting point of the local dose modification is a preliminary treatment plan leading to a preliminary dose distribution in the target and an adjacent structure comprising healthy tissue and/or organs at risk. In the present embodiment, the treatment to be planned comprises IMRT as described above and the treatment plan is represented by $N_b$ beam spots each being subdivided into $b_k$ bixels ($k$ = 1..$N_b$). The bixels may cause a dose distribution which is typical for photons however, the system is not limited to photons but can be employed for any type of therapeutic radiation. If we consider a voxel within the target, there is at least one but generally several bixels from each beam spot influencing the dose in voxel *i*. Assuming an influence matrix $D_{ij}$ representing the dose contribution of bixel *j* on voxel *i*, the physical dose in voxel i is given by:

$$d_i = \sum_{k=1}^{N_b} \sum_{j_k}^{b_k} w_{j_k}^k D_{ij_k}^k \qquad (1)$$

with $w_{j_k}^k$ being the weight of bixel $j_k$ from beam *k*.

Assume that the dose $d_c$ in a specific voxel c has to be changed while the dose in the other voxels should remain as unchanged as possible. This is the simplest form of local dose variation:

$$\tilde{d}_c = d_c + \Delta d_c \qquad (2)$$

This dose variation may be imposed as a hard constraint. To enforce the demanded dose variation, the weights of the bixels influencing the dose of voxel *c* may be changed according to $\Delta d_c$:

$$\tilde{d}_c = \sum_{k=1}^{N_b} \sum_{j_k}^{b_k} w_{j_k}^k D_{cj_k}^k + \Delta d_c \tag{3}$$

$$= \sum_{k=1}^{N_b} \sum_{j_k}^{b_k} (w_{j_k}^k + \Delta w_{j_k}^k) D_{cj_k}^k = \sum_{k=1}^{N_b} \sum_{j_k}^{b_k} \tilde{w}_{j_k}^k D_{cj_k}^k \tag{4}$$

where $\tilde{w}_{j_k}^k$ are the new weights ensuring the required dose variation $\Delta d_c$. The values for $\tilde{w}_{j_k}^k$ can be determined in various ways, as long as the condition

$$\sum_{k=1}^{N_b} \sum_{j_k}^{b_k} (\Delta w_{j_k}^k) D_{cj_k}^k - \Delta d_c = 0 \tag{5}$$

is fulfilled.

One intuitive way is to distribute the "burden" of changing the dose evenly on all participating bixels, i.e.

$$\tilde{d}_c = d_c(1 + \frac{\Delta d_c}{d_c}) = \sum_{k=1}^{N_b} \sum_{j_k}^{b_k} w_{j_k}^k D_{cj_k}^k (1 + \frac{\Delta d_c}{d_c}) \tag{6}$$

$$= \sum_{k=1}^{N_b} \sum_{j_k}^{b_k} \tilde{w}_{j_k}^k D_{cj_k}^k \tag{7}$$

which implies $\tilde{w}_{j_k}^k = w_{j_k}^k (1 + \frac{\Delta d_c}{d_c})$. This distribution of the weight changes is depicted in Figure 2a. The relative change in all the weights of the involved bixels equals the relative dose variation of voxel c:

$$\frac{\Delta w_{j_k}^k}{w_{j_k}^k} = \frac{\Delta d_c}{d_c} \tag{8}$$

The effect of this simple scaling technique on the dose contribution to voxel c is shown in Figure 2b assuming one 1-dimensional beam field $l$, $l \in l..N_b$. Since in the following only one beam field is considered, the index $l$ is not needed and the abbreviation $j \equiv j_l$, $w_j \equiv w_{j_l}^l$ and $D^l \equiv D$ is used. The ordinate of Figure 2b specifies the change in the physical dose of the contributing bixel $j$ to voxel $c$ relative to the current weight of the bixels. The weights are assumed to be equal for all bixels of beam $l$. The lateral profile of one bixel is assumed to be similar to a typical photon profile which is

plotted in solid lines as a reference for the bixel $j^d$ which directly hits voxel *c*. Using the abbreviation $\Delta d_{cj} = \Delta w^l_{j_l} D^l_{cj_l}$, the values connected through the dashed lines describe the relative change $\frac{\Delta d_{cj}}{w_0}$ in the dose contribution to voxel *c* from bixel *j*. It stands to reason that $\frac{\Delta d_{cj^d}}{w_0}$ is maximal because $j^d$ hits voxel c directly and contributes the most dose among the bixels of the radiation field. A bixel with its central axis passing by farther from voxel *c* (e.g. the first bixel $j^l$ of the beam line influencing voxel *c*) does not contribute much dose variation to c due to the decreasing penumbra of the lateral photon profile. Although this strategy is easy to apply, the downside of this method is that unwanted dose deviations occur in many uninvolved voxels within a wide location around voxel *c*. For example a voxel *c*' directly hit by bixel $j^l$ receives a high dose deviation from that bixel while its contribution to voxel c is only small. This is due to the fact that the weight of all bixels are scaled the same way, no matter how much dose they contribute to the considered voxel *c*. Consequently, the dose change $\Delta d_c$ is not only imposed on voxel *c*, but on every voxel which is also influenced by bixels $[j^1 \ldots j^{max}]$ contributing dose to *c* thus introducing big dose deviations in a significant number of uninvolved voxels.

Another possibility to enforce the dose variation in voxel *c* is to adapt the weight of only one bixel per beam, where the bixel which contributes the most dose to voxel c is chosen, i.e. the bixel $j^d$ having central axis closest to c. Thus, $\Delta w_j = 0 \;\forall\, j \neq j^d$. The relative change of bixel weight $w_{jd}$ is derived from condition (5):

$$\frac{\Delta w_{j^d}}{w_{j^d}} = \frac{\Delta d_c}{D_{cj^d} \cdot w_{j^d}} = \frac{\Delta d_c}{d_{cj^d}} \tag{9}$$

The result is depicted in Figure 3. It shows figurative that all the burden of the dose variation $\Delta d_c$ lies on the dose contribution $D_{cjd}$ of bixel $j^d$. Therefore, the spreading of the relative changing dose distribution narrows down to only one bixel depicted in Figure 3b. This method has the advantage that the dose of as few voxels as possible is changed.

In practice, mixtures between these extreme approaches can be employed. For example, for every beam spot, a subset of bixels may be considered having a relative contribution to the local dose which exceeds a predetermined threshold.

<u>Step 2: Dose recovery for affected voxels, whose dose should remain unchanged</u>

Naturally, the variation achieved in voxel c causes unwanted dose changes in a set of voxels *c*' that are exposed to bixels with the modified weights $\tilde{w}^k_{j_k}$. Due to the linear superposition of the contributions from the bixels, the physical dose of voxels *c*' can be separated into two fractions, i.e.,

$$\tilde{d}_{c'} = \sum_{k=1}^{N_b} \sum_{j_k=1}^{u_k} \tilde{w}^k_{j_k} D_{c' j_k} + \sum_{k=1}^{N_b} \sum_{\lambda_k=u_k+1}^{b_k} \tilde{w}^k_{\lambda_k} D_{c' \lambda_k} \tag{10}$$

The first term involves bixels that also contribute dose to voxel *c* while the bixels $\lambda_k$ from the second term only contribute dose to *c*' but not to *c*. Herein a reordering of the bixels was carried out to separate the dose contributions.

The $u_k$ weights $\tilde{w}^k_{j_k}$ of the first term have been previously adapted in order to enforce the desired local dose variation. Their values are not to be changed in the second step of our strategy, since the dose variation shall not be affected by the recovery process. The bixels $\lambda_k$ do not contribute any dose to voxel *c*. The central axis of these bixels is too far away from voxel *c*, so that the lateral profile vanishes before c is reached.

It is the aim of the dose recovery to find an appropriate set $\{\lambda_k\}$ of free bixel weights which results in a sufficient recovery of uninvolved voxels *c*'. This process is supposed to be made in real-time. Therefore, the conventional search

strategy is not feasible. The idea is to restore the dose in a subset of voxels $c'$ in a similar way like the dose variation was done for voxel c. Now, the desired dose restoration $\Delta d_c$, has the opposite sign and is generally smaller than $\Delta d_c$.

Data Structure

In order to efficiently carry out the solution for the local dose shaping problem on a computer, in the preferred embodiment a modem object-operated software design was created. The main data structure of the local dose shaping implementation is summarized in Fig. 4.

The elements shown in the left box of Fig. 4 are used to provide a graphical user interface to the therapist and to present the results of the local dose shaping. For these elements, the data size is not very critical. As shown in the left box of Fig. 4, the components involved are as follows:

- A "dose-array" and a "reference-array" resembling the treatment volume with a first resolution which is the higher resolution. Each of the dose-array and reference-array comprises a set of voxels with an associated dose value. The reference-array may store data representing a reference dose distribution according to a preliminary treatment plan or a given dose prescription by the user and the dose-array may store data representing a dose distribution according to a revised treatment plan.

- A class "VoiPoznt" holding contour data that was produced prior to the therapy planning by a physician to separate the tumor tissue from the healthy tissue. The data consists of several points to mark the boundary of a volume of interest (VOI). Based on these points, there is a fast algorithm in the class VoiPoint that resolves the classification of a voxel to a volume of interest. Accordingly, such classification has not to be stored in the dose-array, which allows to save a tremendous amount of memory which in turn allows for a faster planning.

- A class "Bitmap" holding a medical image such as a CT image which may be used to update a pictographic representation of the dose shaping results.

The elements shown in the right box of Fig. 4 provide the data structures necessary for the recovery process. They are designed to consume as little memory as possible such as to be small enough to reside in the higher memory during the actual dose shaping. This way, the effect of the von-Neumann bottleneck, according to which the data transportation is a limiting factor for performance, can be reduced.

The dose-grid and the reference-grid shown in the right box of Fig. 4 comprise pre-selected subsets of voxels which are potential candidates for a recovery process. In the preferred embodiment, the dose/reference-grids are chosen to be equally distanced grids overlaying the dose-array/reference-array shown in the left half of Fig. 4 at a second, lower resolution as compared with the dose/reference-array. The reason for introducing the dose/reference-grid concept is to reduce the amount of data on which the algorithm is operating such as to save valuable cache memory.

The concept of the dose-grid is illustrated in Fig. 5. Fig. 5b shows a phantom of a target 10 surrounding an organ at risk (OAR) 12. Between the target 10 and the OAR 12, healthy tissue 14 is located. The dose-grid is represented by the points shown in Fig. 5b. As can be seen from Fig. 5b, the dose-grid has sub-grids with different lattice spacings depending on the tissue class. Namely, as can be seen from Figs. 5a and 5b, the sub-grid corresponding to the healthy tissue 14 has the largest lattice spacing $x_h$, while the OAR 12 has the smallest lattice spacing $X_{OAR}$. The lattice *spacing* $x_t$ of the sub-grid corresponding to the target is inbetween. To consider voxels where important dose gradients are expected, each sub-grid preferably starts at the tissue class boundary. This way, the voxel density at the edges of the target- and OAR-edges is higher, which increases the chance for an accurate dose recovery in these regions.

The sub-grid for the healthy tissue has the widest mesh, because most of the healthy tissue is located outside the planning scope near the border of the setup. Here, only a few bixels contribute dose, and the density of bixel intersection points is low. However, the healthy tissue mesh is necessary to scan for hot spots which might occur in these regions. By making the healthy tissue mesh wider than the other, the planning scope may be shifted to the target and the OARs.

Fig. 5c symbolically represents the memory structure for the dose-grid. The voxels on the nodes of the grid are stored adjacently in row order as shown in Fig. 5c. Besides being smaller than the dose-array, the dose-grid structure has the further advantage that the relevant voxels for the recovery algorithm are much closer together in the memory. On the one hand, unnecessary voxels from the target and OARs have been taken out by choosing a smaller resolution. On the other hand, voxels from healthy tissue which are located between the OARs sub-grid and the target sub-grid can be significantly reduced in number. This can be seen for instance by comparing the space $d_l$ in Fig. 5b with the corresponding memory distance $d_l'$ represented in Fig. 5c.

With reference again to Fig. 4, the class dose-deposition comprises a few radiation specific parameters and arithmetic instructions to calculate a desired dose deposited by any bixel. The dose-deposition for example allows to

calculate a dose contribution of a bixel *j* in a depth *d* and a distance *x* away from the central axis of the bixel, and thus allows to obtain information corresponding to the information that was contained in the influence matrix *D* shown in equation (1). The reason for storing the dose-deposition-class instead of a pre-calculated influence matrix is that the data size of the latter would be much too high to reside in higher cache levels at all time, thus forbidding a real-time dose recovery calculation due to the von-Neumann bottleneck.

Due to the use of the dose-grid-concept and the dose-deposition-class, the recovery uses only a small amount of memory while the performance load is shifted to the arithmetic components of the planning computer.

Work Flow of Local Dose Shaping

With reference to Fig. 6, the work flow of the local dose shaping process according to an embodiment of the invention will be summarized. The dose shaping starts with step 16 of applying a local dose variation. According to a preferred embodiment, this is done interactively using a graphical user interface described in detail below. After setting up a dose-grid as shown in Fig. 5 (step 18), the actual recovery process framed by a dashed square 20 in Fig. 6 begins to compensate the dose variation for "uninvolved voxels.". Herein, "uninvolved voxels" are voxels for with the dose was not meant to be changed due to the local dose variation. The "uninvolved voxels" are also said to be in a "predetermined recovery area".

According to the dose recovery process 20, in a first step 22, a voxel c' to be recovered is selected according to a predetermined selection strategy S symbolically represented by bubble 24 in Fig. 6. After choosing a given voxel *c*', a single dose recovery step 26 is performed according to a predetermined recover strategy *R* symbolically represented by bubble 28 in Fig. 6.

The result of the recovery step 26 is a revised set of weights for the bixels, i.e. a revised treatment plan. Using the adjusted weights, a dose calculation on the dose-grid is performed to evaluate the impact of the weight alterations on the whole plan. The dose distribution on the dose-grid is updated, and it is checked whether a predetermined stop criterion is met. If the stop criterion is met, the dose recovery 20 is terminated. In the alternative, the dose recovery cycle repeats at step 22 again, although based on the updated dose-grid.

The system of the invention is not limited to any specific selection strategy *S* (see bubble 24 in Fig. 6). A simple and intuitive selection strategy is to select the voxel which has the largest deviation from the desired dose value. While searching for the voxel of maximal dose deviation may seem to be a time critical process, the run time for searching is in fact not significant, because the dose-grid is small enough to reside in the higher memory, such that data can be rapidly assessed by the arithmetic unit that performs the fast comparison on the dose data.

As regards the recover strategy *R*, the recovery processes in principle are carried out in the same way as the variation process described above, but in opposite direction. However, while for the local dose variation, it is usually acceptable or even desirable that the dose in the vicinity of a selected voxel is also adjusted in a similar way, with regard to the dose recovery, it is generally preferred that a recovery of a voxel c' stays as local as possible. In other words, it is preferred that one recovery step only imposes a small and preferably localized change to the current dose distribution. A good distribution will then be obtained as a result of many local steps involving several voxels to be recovered. This "adiabatic" character of the recovery process has been found to stabilize the local dose shaping by giving the selection strategy the opportunity to find several appropriate voxels. The recovery of these voxels results in a diversity of compensating spots which all contribute their most eligible influence to the resulting plan.

In order to keep the recovery of a voxel c' local, in a preferred embodiment only one bixel $\sigma_k$ that contributes most to the dose of the voxel c' is considered from each beam *k*.

Further, it may occur that the recovery of a voxel prefers one incident beam direction over another. For example, one bixel direction can influence the voxel to recover first handed, while bixels from other directions have to traverse a wide area of the plan to reach the desired voxel. It is also possible that a sensitive organ or an area that is object to another planning aim lies within the path of the bixel. In these cases, it is preferable that the recovery strategy distributes the changing of the weights unequally among the considered bixels according to their relevance. This concept shall be explained in more detail with reference to Fig 7.

Fig. 7 shows the path of given incident bixels $\sigma_k$ which have been chosen to participate in the recovery of the voxel 32. The solid lines shown in Fig. 7 represent the central axes of the participating bixels. Note that the central axes do not have to meet exactly in the same point, since the bixels themselves have a certain width (10 mm in the present embodiment), such that there is not always one bixel from every direction hitting the voxel directly.

As can be seen from Fig. 7, some of the bixels are more appropriate for recovering the dose in the marked voxel 32 than others. The path of bixel $\sigma_1$ for example has a relatively large overlap with the target tissue of the setup. Reducing the weight of bixel $\sigma_1$ would result in a "cold channel" along its path influencing a large number of target voxels. In contrast to this, bixel $\sigma_3$ would only influence a small fraction of the target structure. This beam direction is therefore more appropriate to impose a dose difference to the desired voxel 32 while the rest of the plan changes as little as possible. In a preferred embodiment, the recovery strategy considers these geometrical peculiarities when calculating

the changing weights of the bixels $\sigma_k$. In other words, the changing of the weights $\Delta w_{\sigma_k}$ is not equal for all $k$.

In one embodiment, the ansatz for determining the weight changing $\Delta w_{\sigma k}$ of a bixel $\sigma_k$ that is considered for the recovery process is:

$$\frac{\Delta d_{c'}}{|k|} = D_{c'\sigma_k} \Delta w_{\sigma_k} \qquad (11)$$

with $\Delta d_c$ being the dose difference to be restored in voxel $c'$. $|k|$ denotes the number of bixels (number of beams, if $c'$ is located in the target) which are considered for the recovery process. $D_{c'\sigma_k}$ is the dose contribution of bixel $\sigma_k$ to voxel $c'$. Please bear in mind, that the value of $D_{c'\sigma_k}$ is not read in from the memory. It is merely a mathematic expression which refers to the concept of the influence matrix. Its value is calculated on the fly in the dose-deposition class.

To consider the plan geometry for the recovery process, some incident beam direction may be preferred over another. To determine the impact of one bixel $\sigma_k$ on the plan, a score $f_{\sigma_k}^{c'}$ may be assigned to each bixel:

$$f_{\sigma_k}^{c'} = D_{c'\sigma_k} g_{\sigma_k} \qquad (12)$$

The geometry factor $g_{\sigma_k}$ estimates the impact of changing the weight of the bixel:

$$g_{\sigma_k} = \begin{cases} \dfrac{1}{t_{\sigma_k} + h_{\sigma_k}} & \Delta d_{c'} > 0 \\ \dfrac{1}{t_{\sigma_k} - h_{\sigma_k}} & \Delta d_{c'} < 0 \end{cases}$$

Herein, $t_{\sigma_k}$ and $h_{\sigma_k}$ represent the number of voxels from the target and from the healthy tissue which are influenced by bixel $\sigma_k$ on its path through the plan geometry. If $\Delta d_{c'} > 0$, i.e. a cold spot around the voxel to recover $c'$ has to be compensated, target voxels and healthy tissue voxels are compromised. The weights of the bixels $\sigma_k$ have to be incremented, so unwanted higher dose along the bixel path emerge. If a hot spot around $c'$ must be compensated ($\Delta d_{c'} < 0$), the weight of bixels $\sigma_k$ is lowered. A lower dose along the bixel track is unfavorable for the target dose distribution but of virtue for the healthy tissue. Thus, the number of voxels from healthy tissue is subtracted from the impact.

The score $f$ helps to weight the contribution of the incident beam direction in equation (11). The normalized ansatz used for the changing of the bixel weight gets:

$$\Delta w_{\sigma_k} = \frac{g_{\sigma_k} \cdot \Delta d_{c'}}{|k| \sum_{k'} (D_{c'\sigma_{k'}} \cdot g_{\sigma_{k'}})} \qquad (13)$$

while $k'$ runs over all beam fields.

Applying $\Delta w_{\sigma_k}$ according to this formula would completely recover the dose deviation in voxel $c'$. Due to the fact that only one bixel per beam takes part in the recovery, the weight of these $|k|$ bixels would change significantly and produce high dose gradients along the bixel path. However, as discussed above, an adiabatic character in recovering voxel c' is desired to control the convergence speed and the smoothness of the resulting plan. For this reason an adiabatic factor $p$ is introduced. The value of $p$ depends on the number of voxels (ny) which are considered for the recovery process. Furthermore, the smoothness depends on the number of bixels $|k|$ that share the burden of recovering voxel $c'$:

$$\Delta w_{\sigma_k} = \frac{g_{\sigma_k} \cdot d_{c'}}{\sum_{k'} (D_{c'\sigma_{k'}} \cdot g_{\sigma_{k'}})} \cdot p(|k|, n_y) \tag{14}$$

If a small value is assigned to p, the convergence of the recovery process is slow. Recovering one voxel c' results in a smaller change of the bixel weights $\Delta w_{\sigma_k}$. Thus, in order to compensate the impact of the dose variation, more recovery steps are needed. The advantage of a smaller *p* is that the recovery of one voxel only has a small influence on the plan quality and the selection strategy can chose voxels of a broader variety to compensate the dose variation. The steps are smoother while the selection strategy has more control over the whole process.

Now, we can calculate the geometrical impact for the recovery process depicted in Figure 7 due to the model defined in equation (14). The geometry factors $g_{\sigma_k}$ are assigned to the values listed below. The values are normalized for reason of clarity. The contribution of bixels 2

| | $k=0$ | $k=1$ | $k=2$ | $k=3$ | $k=4$ | $k=5$ | $k=6$ |
|---|---|---|---|---|---|---|---|
| $\frac{g_{\sigma_k}}{\sum_{k'} g_{\sigma_{k'}}}$ | 0.16 | 0.12 | 0.18 | 0.17 | 0.12 | 0.11 | 0.14 |

(15)

and 3 are rated higher than for instance the contribution of bixel 4 and 5. This was expected if one considers the graphical representation of the beam path. By reducing the beam weight of bixel 2 and 3, the number of voxels which are exposed to the unwanted dose difference is significantly smaller than for bixels 4 and 5. The adjustment of the individual contribution of every bixel produces smaller unwanted cold spots in the target. Thus, it offers the possibility to reach the same recovery quality in less steps.

Radiation treatment planning system

Having explained the underlying principles of local dose shaping, a specific example of a radiation treatment planning system employing this concept will be described with reference to Fig. 8 to 18.

The radiation treatment planning system of the embodiment comprises a radiation treatment planning computer (not shown) on which a suitable planning software is installed which allows for conducting the local dose shaping. The system further includes a display device (not shown) for displaying a graphical user interface (GUI). Fig. 8 shows a main window 33 of the GUI in which a phantom of a target 10 (a brain tumor in the present example), an organ at risk 12 (OAR, the brain stem in the present example) and surrounding healthy tissue 14 is shown. Note that the phantom geometry corresponds to the one shown in Fig. 5b and Fig. 7.

The exemplary embodiment is directed to a 2-dimensional treatment plan for illustrative purposes. However, a generalization to three dimensions can be made in a straight forward manner and is encompassed by the present invention.

The boundaries of the regions of interest, i.e. tumor 10 and OAR 12 are marked by a number of points 34 held by the class VoiPoint shown in the data structure of Fig. 4. The boundary or contour data was produced prior to the therapy planning by a physician to separate the tumor tissue from the healthy tissue and the tissue of the OAR. As mentioned before, based on these points a fast algorithm in the class VoiPoint resolves the classification of a voxel to a volume of interest, such that this classification does not need to be stored in the dose-array (see Fig. 4).

Also shown in the screenshot of Fig. 8 is a preliminary dose distribution corresponding to a preliminary treatment plan. In the illustration of Fig. 8, the dose distribution is simply represented by a 25 Gy isodose line 36. In the actual GUI, the dose distribution would be represented by a color code which could not be included in the enclosed black and white drawings. Further, a polygon 38 having seven sides is shown, where each side corresponds to one beam direction and is perpendicular to the respective beam axis. The directions of the beams thus correspond to the directions of the selected bixels of the beams shown in Fig. 7. On each side of polygon 38, a number of small circles 40 are shown, of which each represents one bixel. Although this cannot be easily discerned from the illustration of Fig. 8, circles 40 contributing to the planning in the present embodiment are displayed differently (for example by solid lines) than those bixels that do not contribute to the planning (for example shown by broken lines). Namely, bixels that do not cross the tumor region 10 or at least do not come close to it will not be employed in the planning for obvious reasons.

A menue 42 is provided allowing the user to interactively select different shaping tasks. A box 44 can be checked to zoom in such that only the phantom geometry will be shown in the window, as is the case in Fig. 11 to 15.

Also, a box 46 allows to select isodose lines, where in the current setting a 25 Gy isodose line is selected and shown at 36 in Fig. 8. Finally, a box 48 is provided for applying a local dose variation, as will be described in more detail below.

The radiation treatment planning system is a system of interactively shaping the dose in consecutive steps, where in each step a previous or "preliminary" dose distribution according to a corresponding previous or "preliminary" treatment plan is modified or revised. The system may thus start out from a rather sophisticated treatment plan already that has been obtained by a prior art optimization module for inverse treatment planning. In this case, the local dose shaping could be employed only to repair some local deficiencies in an otherwise suitable treatment plan, such deficiencies being for example undesired hot or cold spots or a mismatch of a previous planning with the actual structure of patient geometry which could arise due to a tumor growth between the original planning and the time of therapy. Using the local dose shaping capabilities of the system, these deficiencies can be quickly and interactively accounted for by the radiotherapist immediately before starting the therapy, when there is no time to start a new complete inverse planning based on a prior art optimization module.

However, the local dose shaping may also be employed for developing a treatment plan from scratch, as will be described in the present embodiment. The starting point of the treatment planning is shown in Fig. 9, where an additional window "InfoForm" 50 is illustrated, in which beam profiles 52 for each of the seven treatment beams involved are shown. In each profile 52, the horizontal axis represents the bixel number and the vertical axis represents the intensity or weight of each bixel. As is seen in Fig. 9, all of the bixels have the same intensity or weight, but not all of the bixels participate in the planning, as mentioned before.

When a tab "DVH" is selected in window InfoForm 50, the dose-volume-histogram (DVH) for tumor 10 and OAR 12 can be inspected, as is shown in Fig. 10. Herein, curve 54 represents the DVH for the tumor 10, while curve 56 represents the DVH for the OAR 12. As can be seen from Fig. 10, the dose provided at the tumor 10 will be sufficient, which was to be expected, since all bixels crossing the tumor have been given an equal and sufficient intensity or weight. However, as is also seen from Fig. 10, the dose received by the OAR, while lower than that of the tumor 10 is still inacceptably high. The goal of the treatment planning is thus to modify the profiles 52 shown in Fig. 9 such as to decrease the dose received in the OAR while maintaining the dose in the tumour 10.

With the radiation treatment planning system of the present embodiment, this can be achieved by applying a local dose variation to the OAR 12 such as to reduce the dose therein. As is shown in Fig. 11, the user may select as the simplest type of variation the "one point scheme" by checking the appropriate circle in box 42, as shown in Fig. 11. Next, using an input device such a mouse, the user manually selects a point 58 for applying a local dose variation. Herein, the point 58 is selected right in the center of the OAR 12, i.e. the brain stem. In box 48 at the upper right of GUI 33, the coordinates of the selected points (382, 400) and the dose according to the preliminary treatment plan, 39 Gy, are displayed. By interactively shifting a sliding element 60 of a regulation bar 62, the user can interactively set a new local dose at point 58 to an appropriate value, which is 20 Gy in the present example.

Using the local dose modification method described above, the radiation treatment planning system carries out a local dose variation, which amounts to a revising of the preliminary treatment plan such as to account for the local dose variation inputted by the user. Note that this corresponds to step 16 of the workflow of Fig. 6.

The effect of the local dose variation is shown in Fig. 12, where by the occurrence of the 25 Gy isodose line within the OAR 12 it can be seen that the dose at the selected point 58 and its surrounding has decreased. Note that the new dose value displayed in box 48 is not exactly 20 Gy, but 21,89 Gy. This has to do with a scattering background which was neglected in favor of a more efficient computation such as to minimize the run time.

Note that in the state shown in Fig. 12, the dose recovery has not yet taken place, i.e. the workflow is still outside the dashed box 20 of Fig. 6. It is clear that reducing the dose locally at point 58 will have an influence on the dose distribution in the tumor region 10, although this is not intended. Such unwanted alteration of the dose in the tumor shall be recovered by the dose recovery means of the system, and the region of the tumor 10 thus constitutes a predetermined recovery area, where the dose prior to the local dose variation is to be at least approximately restored.

To understand this in more detail, Fig. 13 shows a screenshot in which the difference of the dose in the tumor region 10 (which is the predetermine recovery area at this occasion) after and prior to the local dose variation is shown. This difference is the difference between the values of the dose-array and reference-array, which with a lower resolution can be represented by the difference of the doses on the dose-grid and the reference-grid according to the data structure of Fig. 4. As can be seen in Fig. 13, due to the local dose variation, unwanted cold spots or cold channels 64, i.e. a local decrease of dose arise in the tumor region. From the shape and orientation of cold channels 64 it can be easily understood that they are due to the reduction of the weight of the voxels passing near the selected point 58, which in turn was caused by the local dose modification. In order to remove the cold spots, i.e. recover the dose within the tumor region 10, the dose recovery scheme explained above and summarized in box 20 in Fig. 6 is carried out.

According to the selection strategy used herein, the voxel of the dose-grid having the largest difference to the reference value is selected and the dose recovery is performed based on this voxel, leading to a revised or updated dose-grid. Based on this revised dose-grid, another voxel is selected according to the section strategy, and the procedure is repeated until a certain stop criterion is met. The stop criterion could for example be a maximum number of cycles or

the meeting of a certain quality standard of the revised dose distribution, for example that the maximum deviation between the reference dose and the dose after recovery is below a certain threshold. In an exemplary embodiment tested by the inventors it was found that for the treatment volume shown in the present embodiment a significant recovery can be reached after ten recovery cycles and that a convergence of the recovery is reached after about 50 cycles, to give a rough estimate.

Fig. 14 shows a screenshot of the dose distribution after the recovery process. Note that the center of the OAR has been kept below 25 Gy in spite of the recovery and that the dose at the selected point 58 is at 20,55 Gy.

Fig. 15 is a similar image as in Fig. 13 showing the difference between the updated dose distribution and the reference dose after the recovery has been carried out. By comparison of Fig. 15 and 13, it can be seen that after the recovery procedure, the cold spots 64 have been largely removed. Note again that in the actual GUIs, the dose distributions would be highlighted according to a color code, so that the dose distribution could be visually understood more easily. Also note that Fig. 12 to 15 show the intermediate steps of the dose shaping, which are displayed for understanding the function of the treatment planning system, but these intermediate steps will not be of interest for the user of the system and will therefore in practice generally not be displayed.

Fig. 16 shows the updated DVH of Fig. 10. In Fig. 16, the dashed lines 54 and 56 show the DVH for the tumor 10 and OAR 12, respectively, prior to applying the dose shaping, and therefore correspond to the solid lines in Fig. 10. Solid lines 54' and 56' show the DVH for the tumor and OAR, respectively, after the dose shaping has been performed. As can be seen in Fig. 16, the DVH for the tumor 10 has practically not changed, as it should be: maintaining the DVH of the tumor 10 is the essential aim of the dose recovery at this instance. However, as is also seen in Fig. 16, the DVH of the OAR 12 has significantly changed, indicating a much smaller dose than before. Note that if the local dose variation had been applied for example at the tumor 12, the predetermined recovery area would then have been the OAR 12 and possibly also the healthy tissue.

Finally, Fig. 17 shows the modified beam profiles after performing the local dose shaping, which is to be compared with the initial constant profiles of Fig. 9. Note that the set of profiles shown in Fig. 12 resembles a suitable treatment plan, as these profiles can be generated by a radiation therapy machine using for example suitable multi-leaf collimators or other suitable techniques.

As can be seen from the screenshots of the GUI of Fig. 8 to 17, using the radiation treatment planning system of the invention, the therapist can interactively adjust the treatment plan by applying a local dose shaping according to his needs and experience. While only a single local dose modification at point 58 has been described, it goes without saying that any number of different points can be selected for dose variation until the dose distribution obtained will suit the therapist's need. Accordingly, the system allows the therapist to interact with the dose shaping system at many stages and "on the fly", since each of the dose shaping steps can be performed in very little time, owing to the data structure shown in Fig. 4 which was devised for optimal speed.

The treatment planning system of the embodiment of the invention is suitable for revising a treatment plan that has been obtained by ordinary inverse planning techniques based on a global optimization according to prior art. However, it can also be used to devise a treatment plan from scratch in a number of consecutive steps allowing the therapist to interact with the system. This is conceptually very different from treatment planning systems known to the inventors, that are based on purely global optimization algorithms.

Finally, it is emphasized that the method of varying or modifying the local dose on a single point basis is only the simplest example, but that other ways of local dose variation may be employed as well. An example is schematically shown in Fig. 18, where the same phantom structure as before is shown. In Fig. 18, a certain isodose line 66 runs right through the OAR 12. Instead of changing the dose distribution by selecting one or more points in the OAR 12 and lowering their dose as described with reference to Fig. 11, in an alternative embodiment, the user can simply shift or drag the isodose line 66 using an input device such as a mouse to a more favorable configuration 66' shown in Fig. 18. This dragging of the isodose line will then be understood by the system as an input of a local dose variation. For example, the system could identify all voxels of a dose-grid lying between isodose curves 66 and 66' and internally perform a dose shaping on these points in a similar way as described before, although without the user having to select all these points individually.

Although the preferred exemplary embodiment is shown and specified in detail in the drawings and the preceding specification, these should by viewed as purely exemplary and not as limiting the invention. It is noted in this regard that only the preferred exemplary embodiments are shown and specified, and all variation and modifications should by protected that presently or in the future lie within the scope of the appended claims.

<u>LIST OF REFERENCE SIGNS</u>

10 target

| | |
|---|---|
| 12 | organ at risk |
| 14 | healthy tissue |
| 16 | local dose variation application step |
| 18 | initialization step dose-grid |
| 20 | dose recovery step |
| 22 | step of selecting a voxel to recover |
| 24 | selection strategy |
| 26 | single dose recovery step |
| 28 | recover strategy |
| 30 | recovery evaluation step |
| 32 | selected voxel for dose recovery |
| 33 | graphical user interface |
| 34 | points marking the boundary of a region of interest |
| 36 | isodose line |
| 38 | polygon indicating the beam directions |
| 40 | point marking a bixel |
| 42 | menue for selecting local beam shaping modality |
| 44 | box for zooming in |
| 46 | box for choosing isodose line |
| 48 | box for inserting a dose value |
| 50 | window showing beam profile |
| 52 | beam profile |
| 54, 54' | dose-volume-histogram for target 10 prior to/after local dose shaping |
| 56, 56' | dose-volume-histogram for organ at risk 12 prior to/after local dose shaping |
| 58 | selected point for applying local dose variation |
| 60 | sliding element for adjusting a dose value |
| 62 | dose regulation bar |
| 64 | cold spot |
| 66, 66' | isodose curve prior to and after shifting in response to user input |

## Claims

**1.** A radiation treatment planning system, comprising:

- means (33) for graphically displaying an image representing a target area (10) to be treated with a set of therapeutic radiation beams, an adjacent structure comprising healthy tissue (14) and/or organs at risk (12), and for displaying corresponding dose values according to an initial or a preliminary treatment plan,
- means (62) for allowing a user to interactively input a local dose variation,
- local dose variation means for revising the initial or preliminary treatment plan such as to account for the local dose variation inputted by the user, and
- dose recovery means comprising means for revising the treatment plan again such as to at least partially compensate for a change of dose in a predetermined recovery area caused by said dose variation.

**2.** The radiation treatment planning system of claim 1,
wherein the treatment to be planned by the system employs therapeutic beams irradiated from different directions and each having a modulated cross-sectional beam intensity profile (52), wherein the modulation of said cross-sectional beam intensity profile may amount to a modulation of the radiation field boundary, a modulation of a uniform intensity of each radiation beam and/or a modulation of the intensity within each radiation field,
wherein said treatment plan comprises data representing a set of cross-sectional beam intensity profiles, one for each irradiation direction, and in particular, a set of bixel-arrays, each bixel array representing a corresponding beam intensity profile (52) in a discretized manner.

**3.** The system of claim 1 or 2, wherein the means for allowing a user to interactively input a local dose variation comprise means for allowing a user to manually select one or more individual points (58) within said image and to change the corresponding dose value, to manually shift an isodose curve (66) displayed in said image using an input device, or to manually shift a graph representing a dose-volume-histogram.

4. The system of one of the preceding claims, wherein the local dose variation means are configured to perform the steps of

- selecting, for every beam, a subset of bixels, and
- adjusting the intensities of the selected bixels by a predetermined mathematical operation insuring an overall change of the local dose related to the inputted local dose variation.

5. The method of claim 4, wherein the selected subset of bixels is formed by a predetermined number of bixels contributing the most to the local dose or by those bixels having a relative contribution to the local dose which exceeds a predetermined threshold.

6. The system of one of the preceding claims, wherein the predetermined recovery area comprises a set of predetermined voxels on which the recovery process is to be carried out, said dose recovery means being configured to

(a) select one of the voxels (32) according to a predetermined selection strategy,
(b) revise the treatment plan such as to at least partially recover the dose at the selected voxel (32), and
(c) compute the revised dose distribution according to the revised treatment plan, wherein steps (a) to (c) are repeated until a predetermined stop criterion is met.

7. The system of claim 6, wherein in step (a) the voxel (32) is selected at least in part based on the absolute value of the dose difference to be compensated or on a combination of said absolute value and the distance of the voxel from the location of local dose variation, and/or
wherein the stop criterion is based on a certain quality of the recovery reached and/or a number of iterations of steps (a) to (c).

8. The system of one of the preceding claims, said system employing a dose-array comprising a first set of voxels resembling the treatment volume with a first resolution, wherein a dose value is assigned to each voxel of said first set of voxels, and wherein said system further employs a dose-grid comprising a second set of voxels resembling the treatment volume with a second resolution lower than said first resolution, wherein a dose value is assigned to each voxel of said second set of voxels, wherein said dose recovery means employ the dose-grid for dose recovery.

9. The system of claim 8, wherein the dose-grid comprises at least two sub-grids having different resolutions, the different resolutions being associated with at least two different tissue classes, said different tissue classes being selected from the group consisting of at least target tissue (10), healthy tissue (14) and tissue of an organ at risk (12).

10. The system of claim 6, wherein in step (b) for every beam one or more bixels contributing most to the dose of the selected voxel (32) among the bixels that had not been involved in the local dose variation is or are selected, and the intensities of each selected bixel are adjusted according to a predetermined mathematical operation ensuring a change of dose at the selected voxel such as to at least approximately recover the original dose.

11. The system of claim 10, wherein, assuming that the selected voxel (32) is located in one of the tissue classes target, healthy tissue and organ at risk, said mathematical operation also accounts for the impact each selected bixel has on the tissue of the remaining two tissue classes.

12. The system of claim 11, wherein said mathematical operation accounts for said impact by accounting for the length along which said bixel traverses tissue of the remaining tissue classes, and in particular,
provided that the recovery at the selected site amounts to a lowering of the dose,
the relative contribution of a bixel in the recovery is increased the longer the transversing lengths through an organ at risk (12) and/or healthy tissue as said remaining tissue class or classes are, and/or
is decreased the longer the transversing length through the target (10) as said remaining tissue class is, and/or
wherein, provided that the recovery at the selected site amounts to an increasing of the dose,
the relative contribution of a bixel in the recovery is decreased the longer the transversing lengths through an organ at risk (12) and/or healthy tissue (14) as the remaining tissue class of classes are.

13. The system of one of the preceding claims, said system being configured to perform, in response to an inputted dose variation, a sequence of alternating local dose variation and dose recovery steps, wherein in each of the local dose variation steps, the local dose variation is performed for a predetermined fraction of the inputted local dose variation value only.

**14.** A computer program product, which when executed by a computer causes the computer to perform the following operations:

- graphically displaying an image representing a target area (10) to be treated with a set of therapeutic radiation beams, an adjacent structure comprising healthy tissue (14) and/or organs at risk (12), and displaying a dose distribution according to an initial or a preliminary treatment plan,
- receiving an input of a local dose variation,
- revising the initial or preliminary treatment plan such as to account for the local dose variation received, and
- revising the treatment plan again such as to at least partially compensate for a change of dose in a predetermined recovery area caused by said dose variation.

**15.** The computer program product according to claim 14, which when installed on a computer materializes a system according to one of claim 1 to 13.

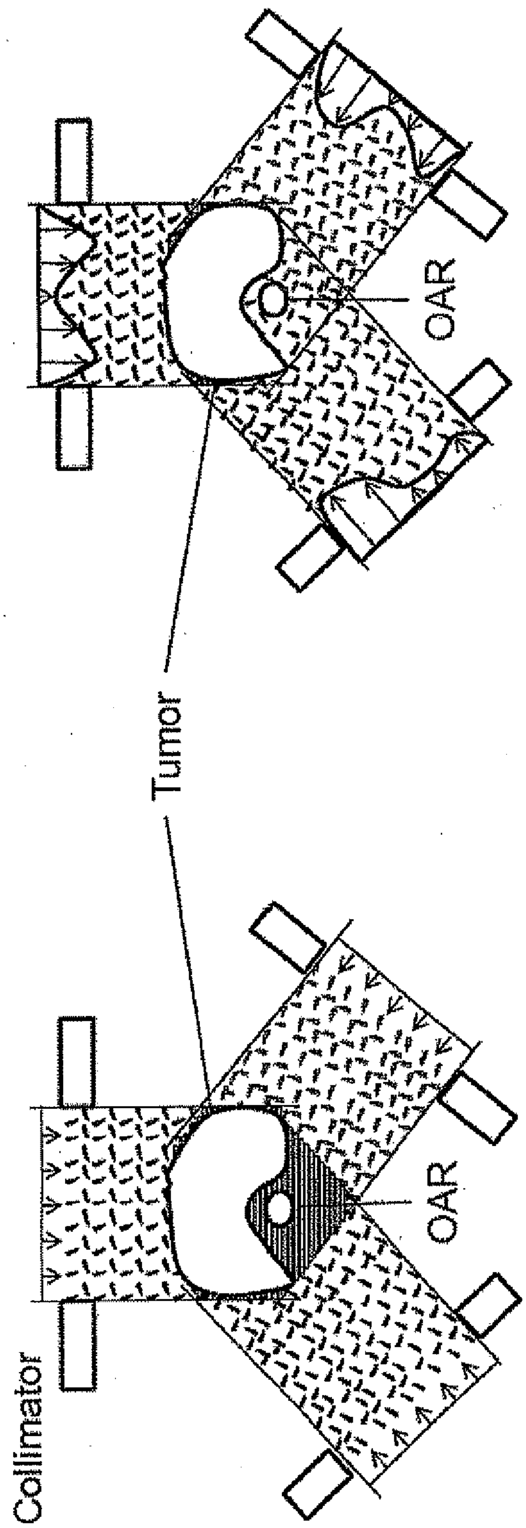


Fig. 1

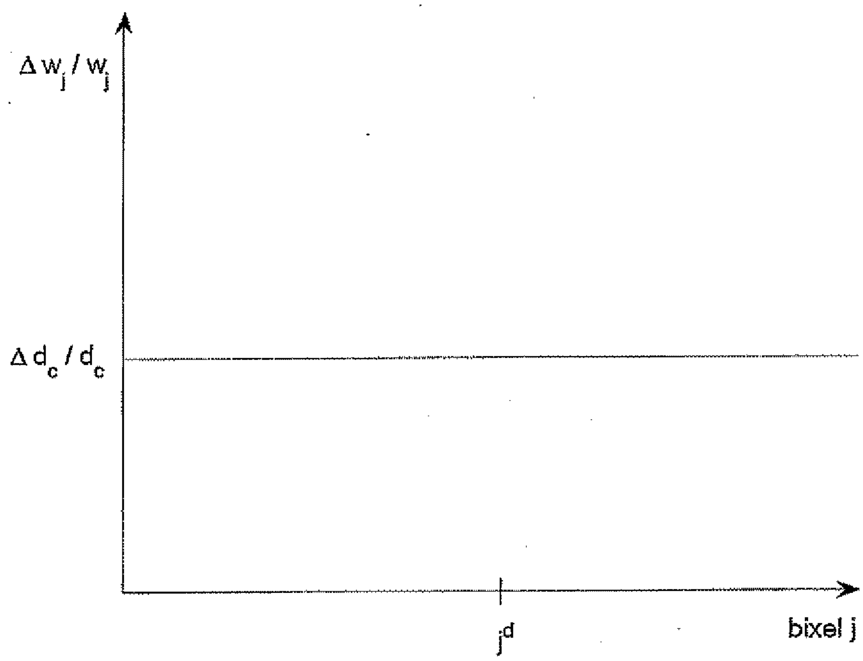


Fig. 2a

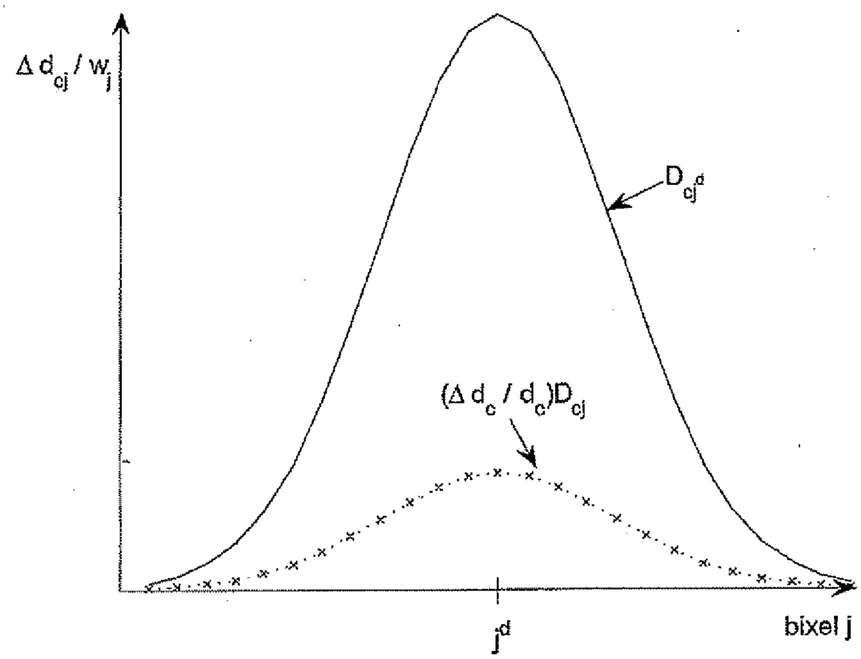


Fig. 2b

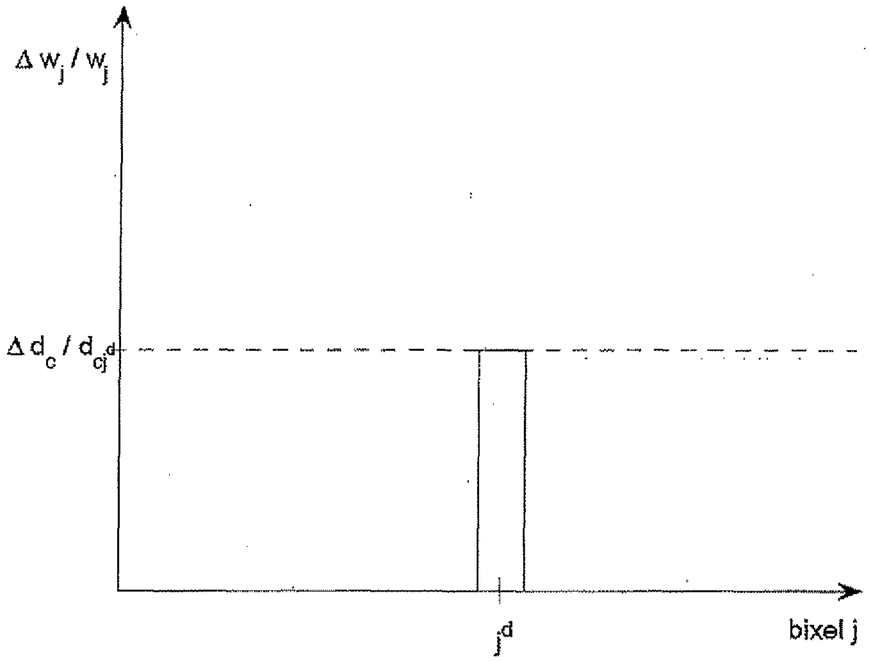


Fig. 3a

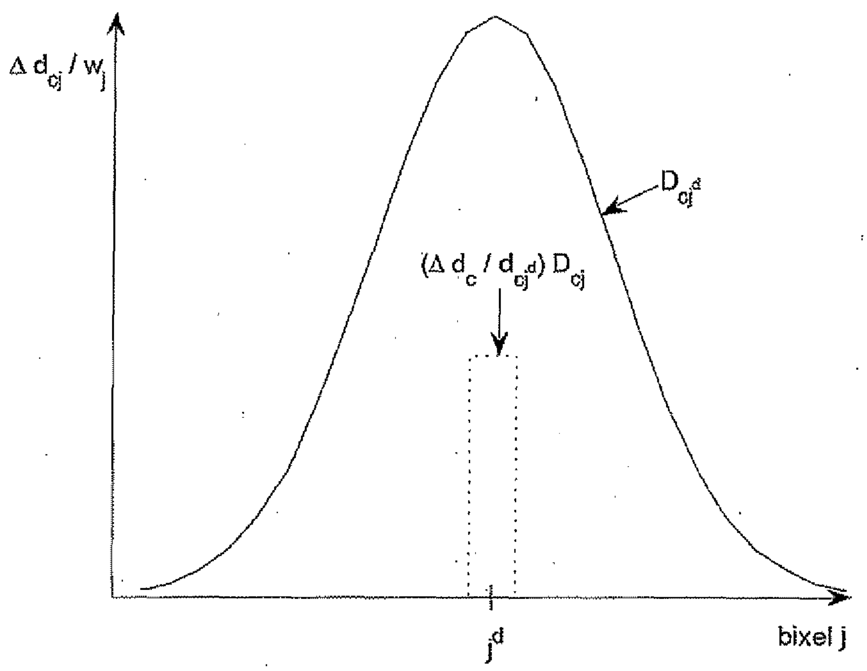


Fig. 3b

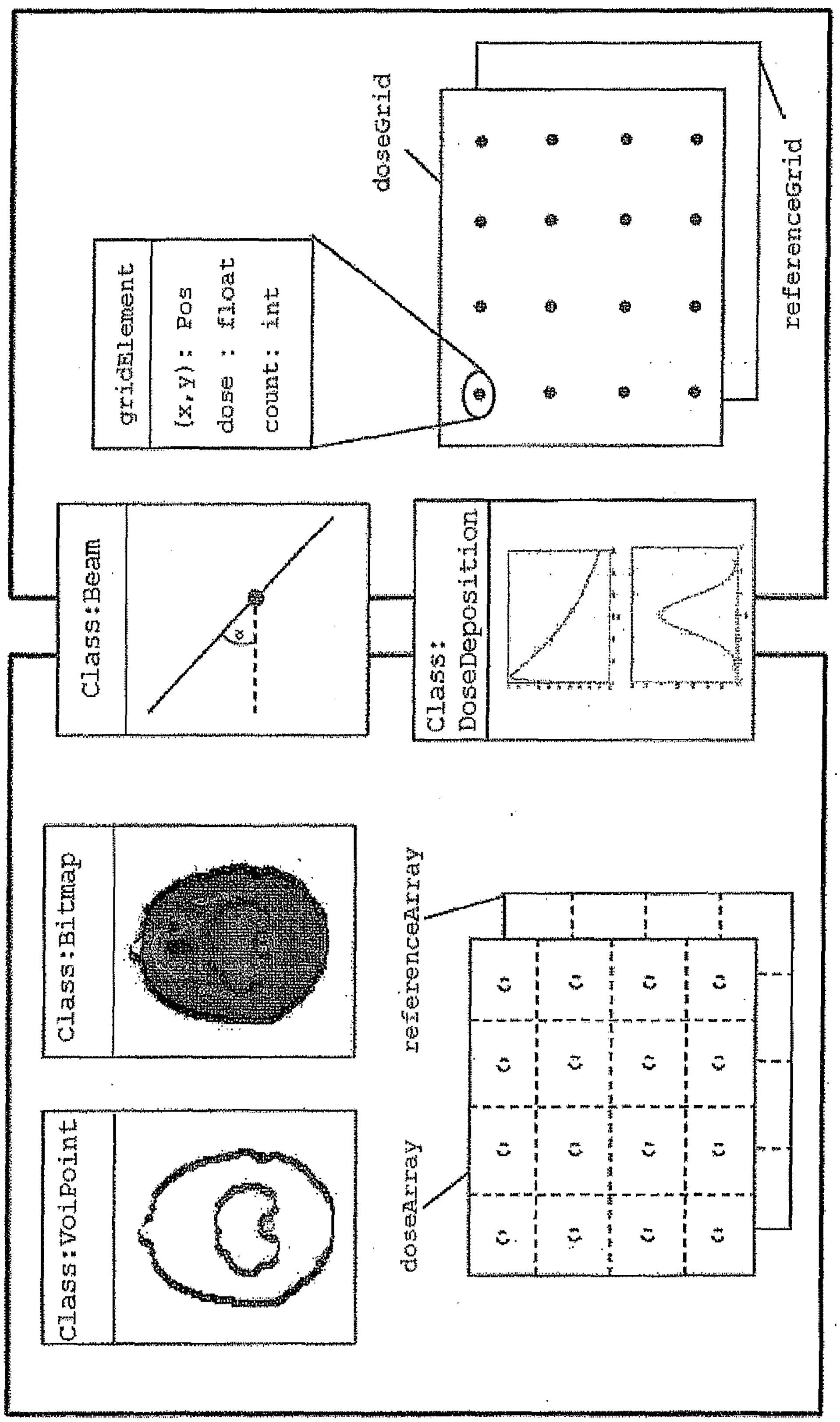
Class:VoiPoint
Class:Bitmap
doseArray
referenceArray
Class:Beam
α
Class:
DoseDeposition
gridElement
(x,y): Pos
dose : float
count: int
doseGrid
referenceGrid

Fig. 4

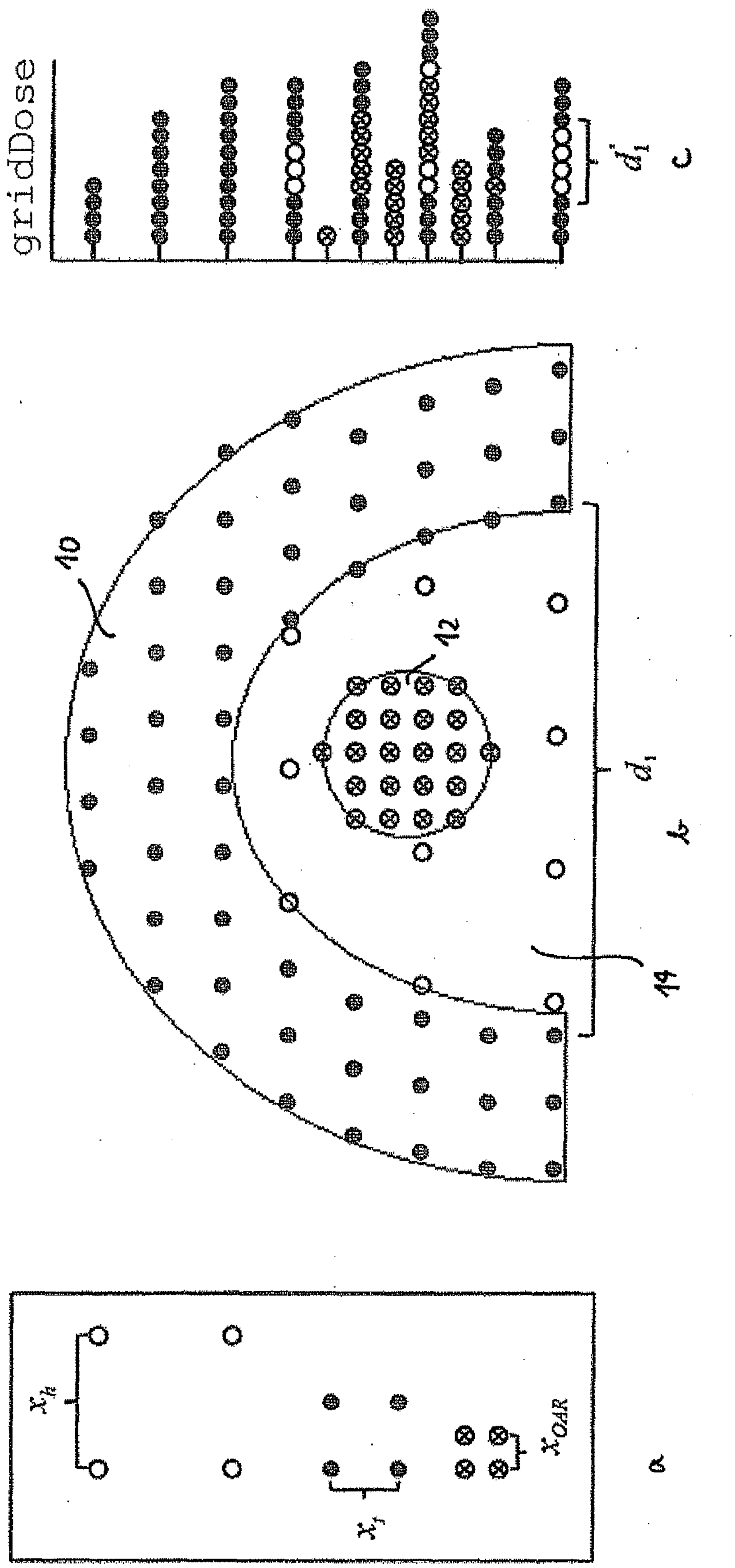


Fig. 5

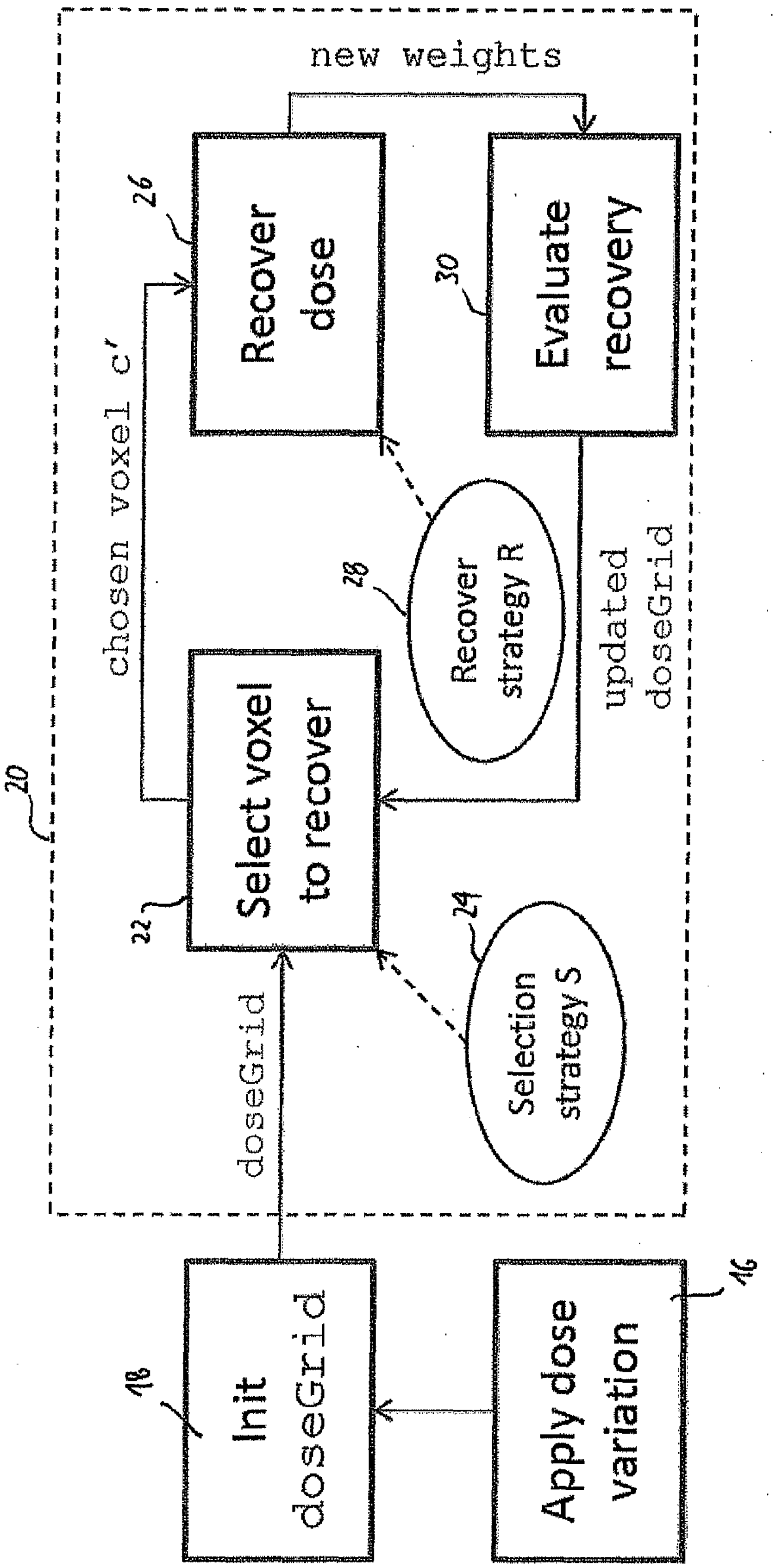
20
Init doseGrid
18
Apply dose variation
16
doseGrid
Select voxel to recover
22
Selection strategy S
24
chosen voxel c′
Recover dose
26
Recover strategy R
28
new weights
Evaluate recovery
30
updated doseGrid

Fig. 6

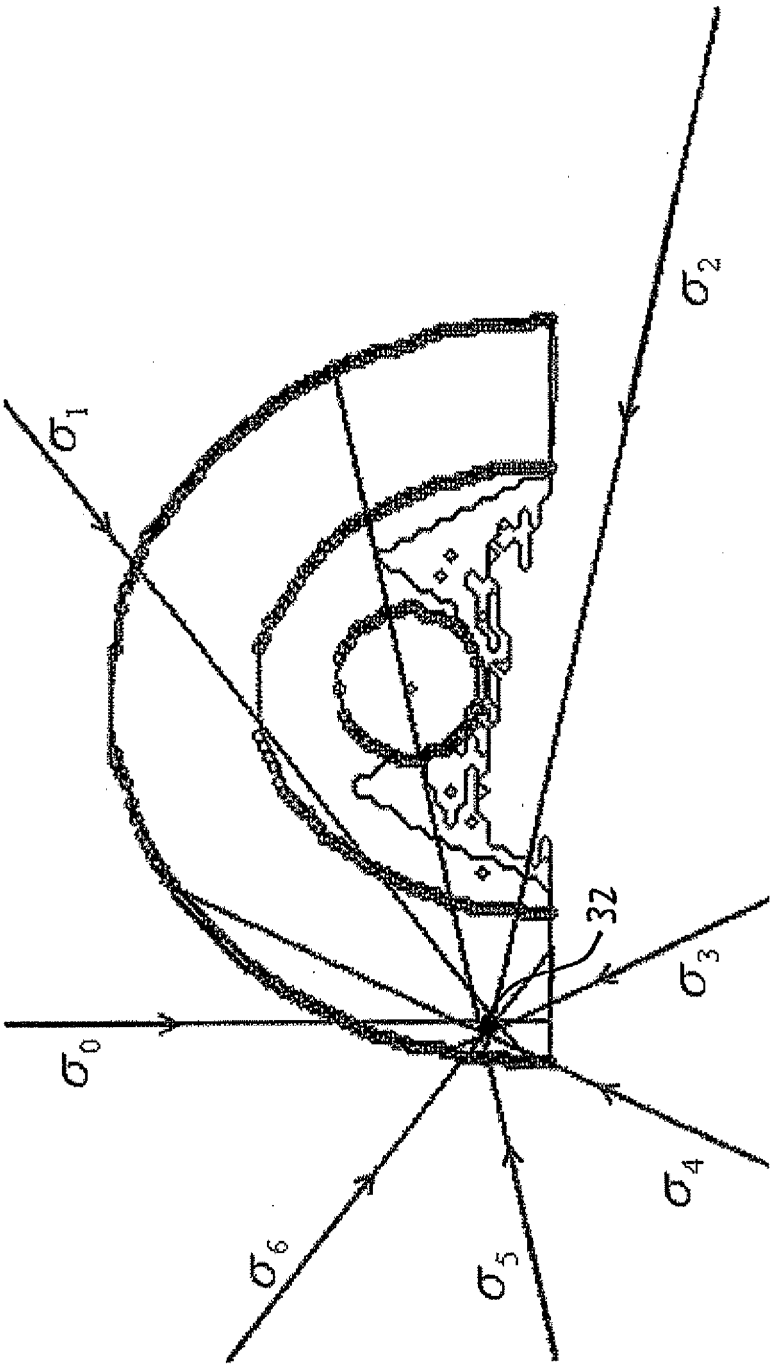


Fig. 7

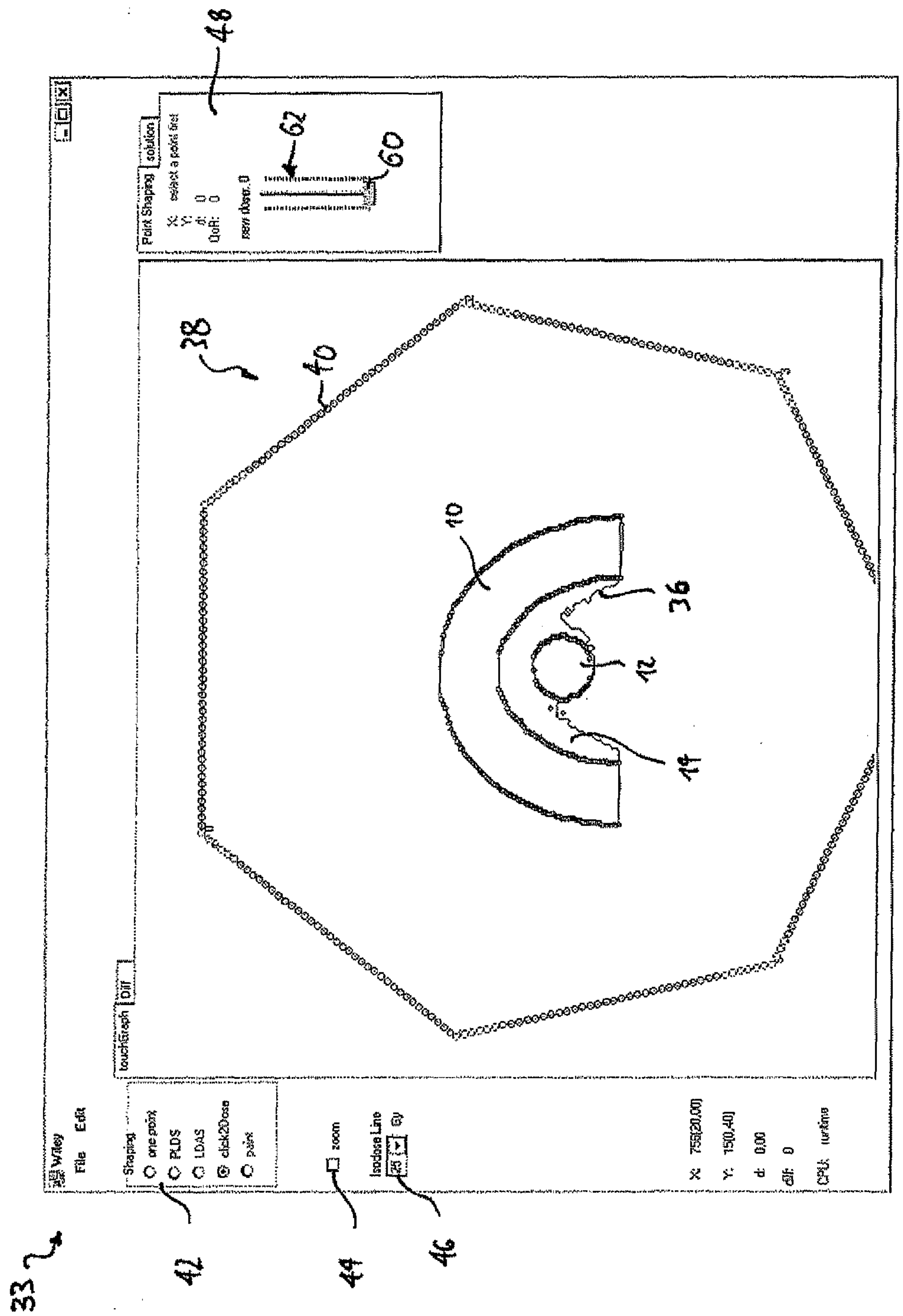
33
42
44
46
48
62
60
38
40
10
36
12
14
File
Edit
Shaping
one point
PLDS
LDAS
zoom
Isodose Line
touchGraph
Diff
Point Shaping
solution

Fig. 8

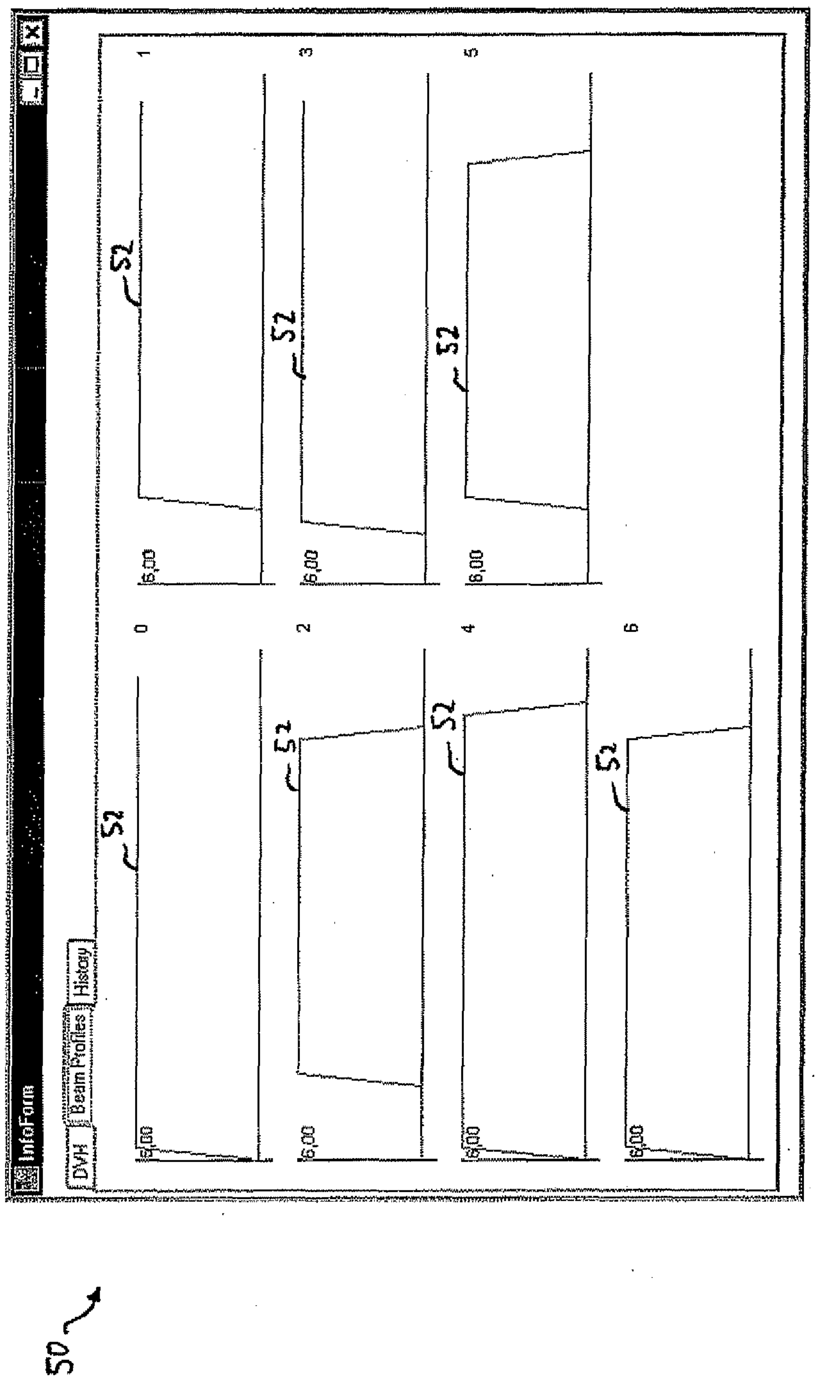


Fig. 9

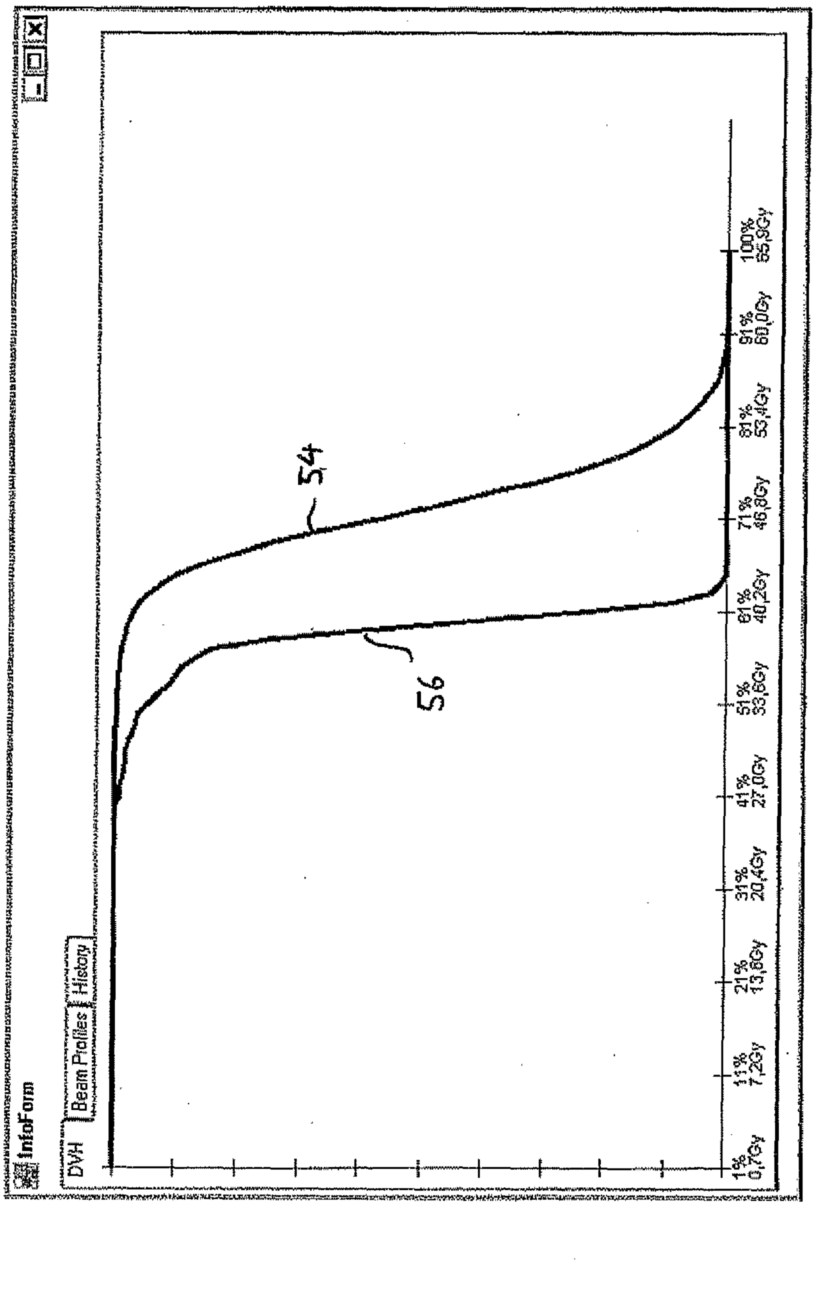
InfoForm
DVH
Beam Profiles
History
1% 0,7Gy
11% 7,2Gy
21% 13,6Gy
31% 20,4Gy
41% 27,0Gy
51% 33,6Gy
61% 40,2Gy
71% 46,8Gy
81% 53,4Gy
91% 60,0Gy
100% 65,9Gy
54
56

Fig. 10

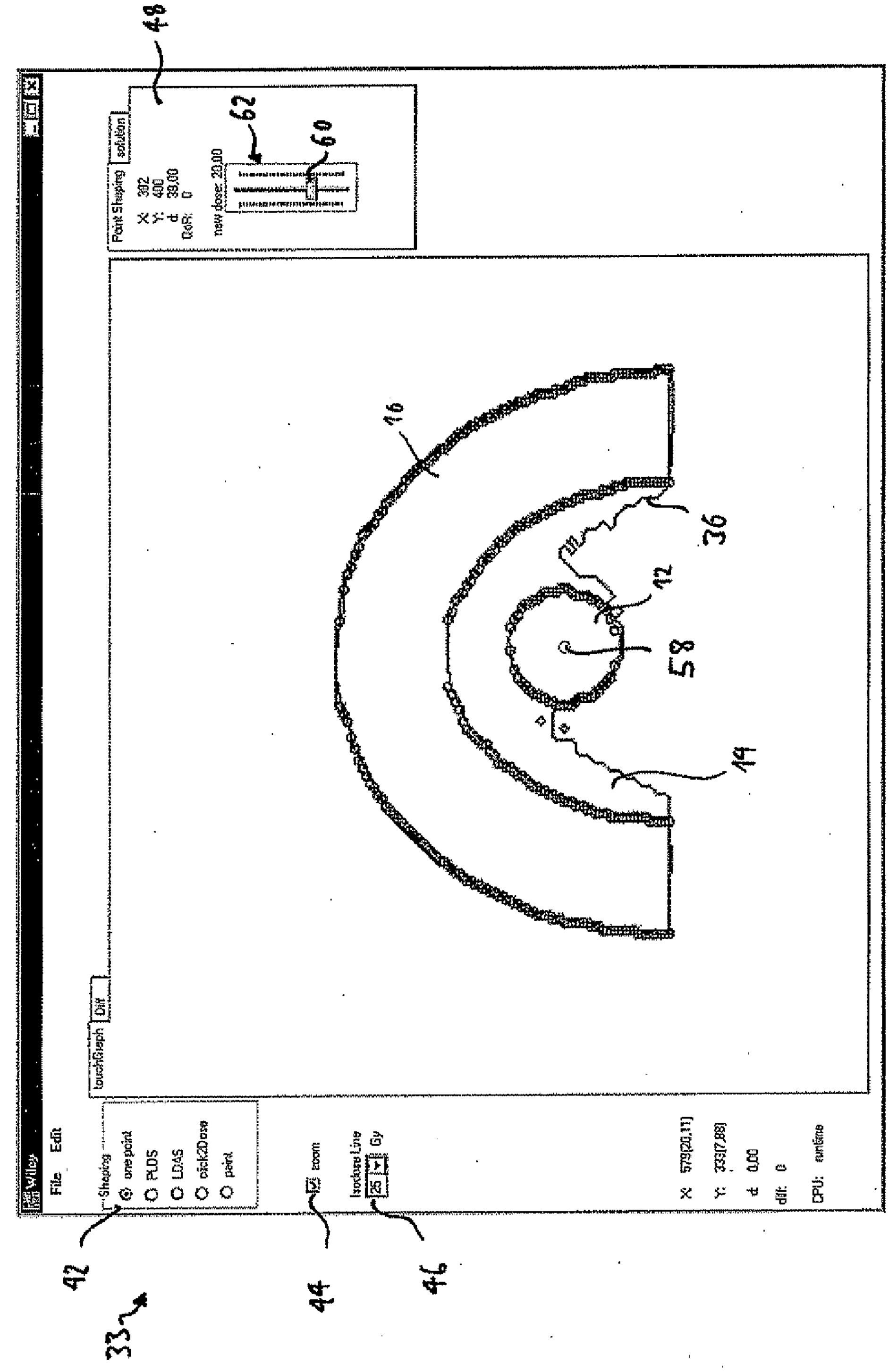
Wiley
File
Edit
Shaping
one point
PLDS
LDAS
click2Dose
paint
zoom
Isodose Line
25
Gy
touchGraph
Dif
Point Shaping
solution
X: 302
Y: 400
d: 39,00
DoR: 0
new dose: 20,00
X: 579[20,11]
Y: 333[7,88]
d: 0,00
dif: 0
CPU: runtime
48
62
60
16
36
12
58
14
42
33
44
46

Fig. 11

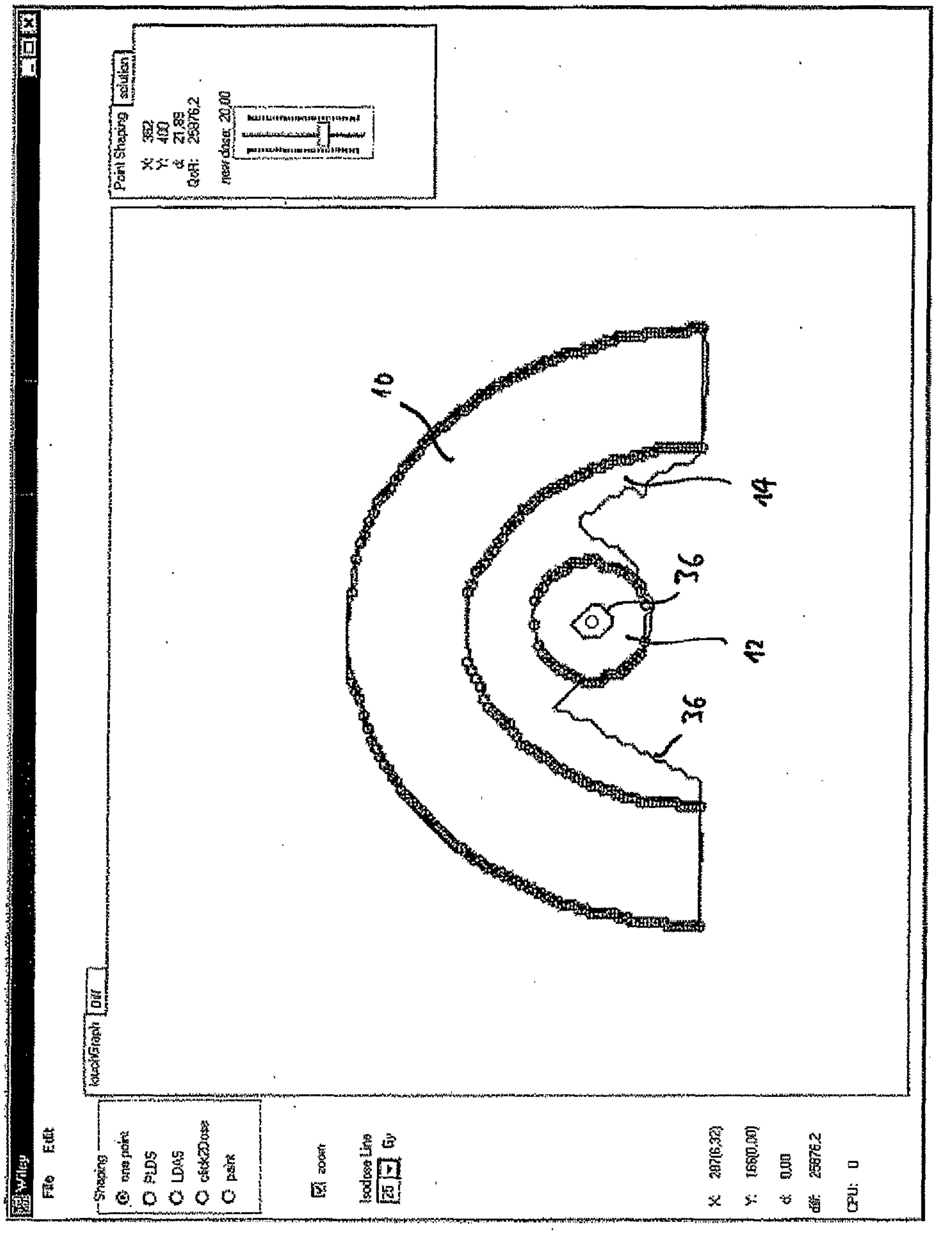


Fig. 12

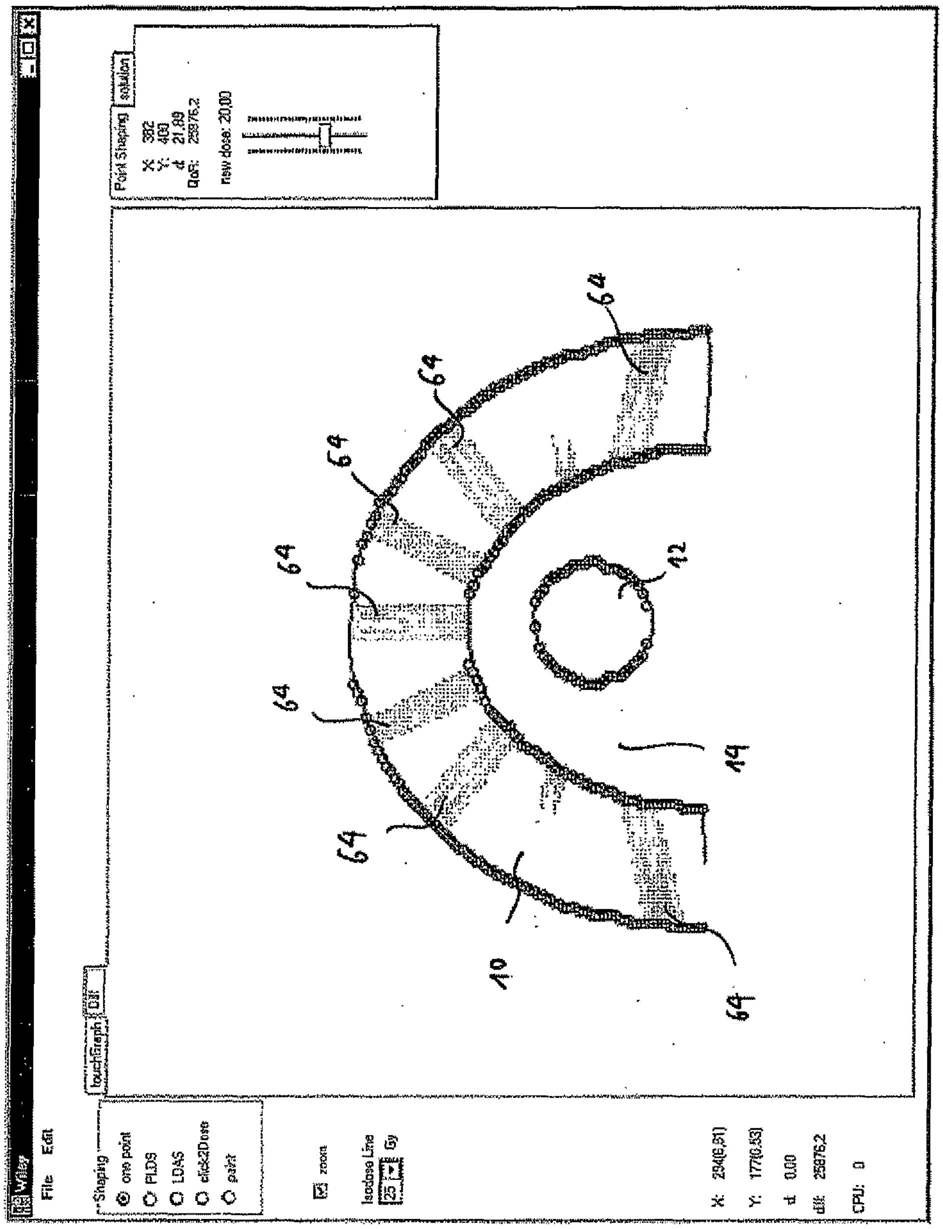
64
64
64
64
64
64
64
64
10
12
14
File Edit
Shaping
one point
PLDS
LDAS
click2Dose
paint
zoom
Isodose Line
Gy
touchGraph
Point Shaping
solution
new dose: 20,00

Fig. 13

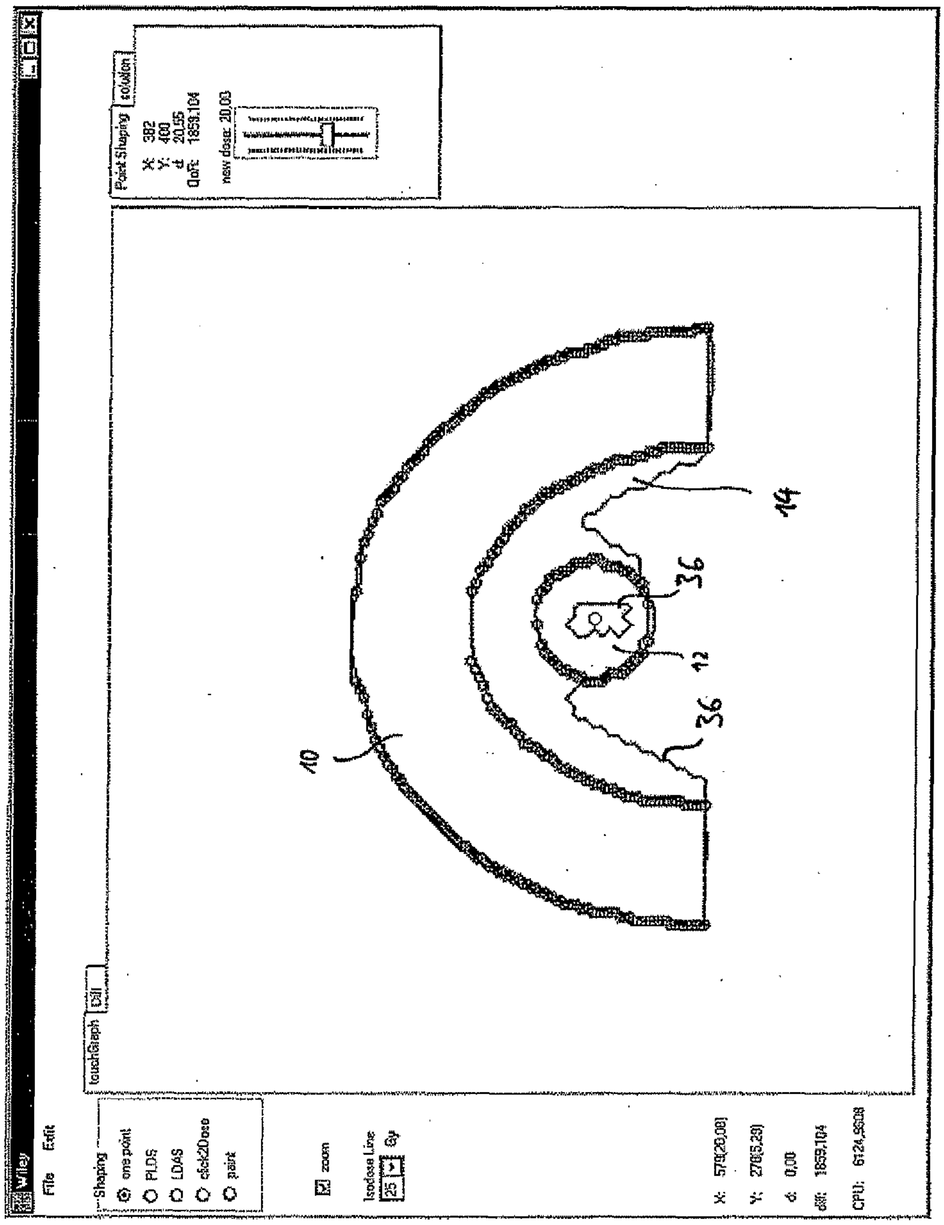


Fig. 14

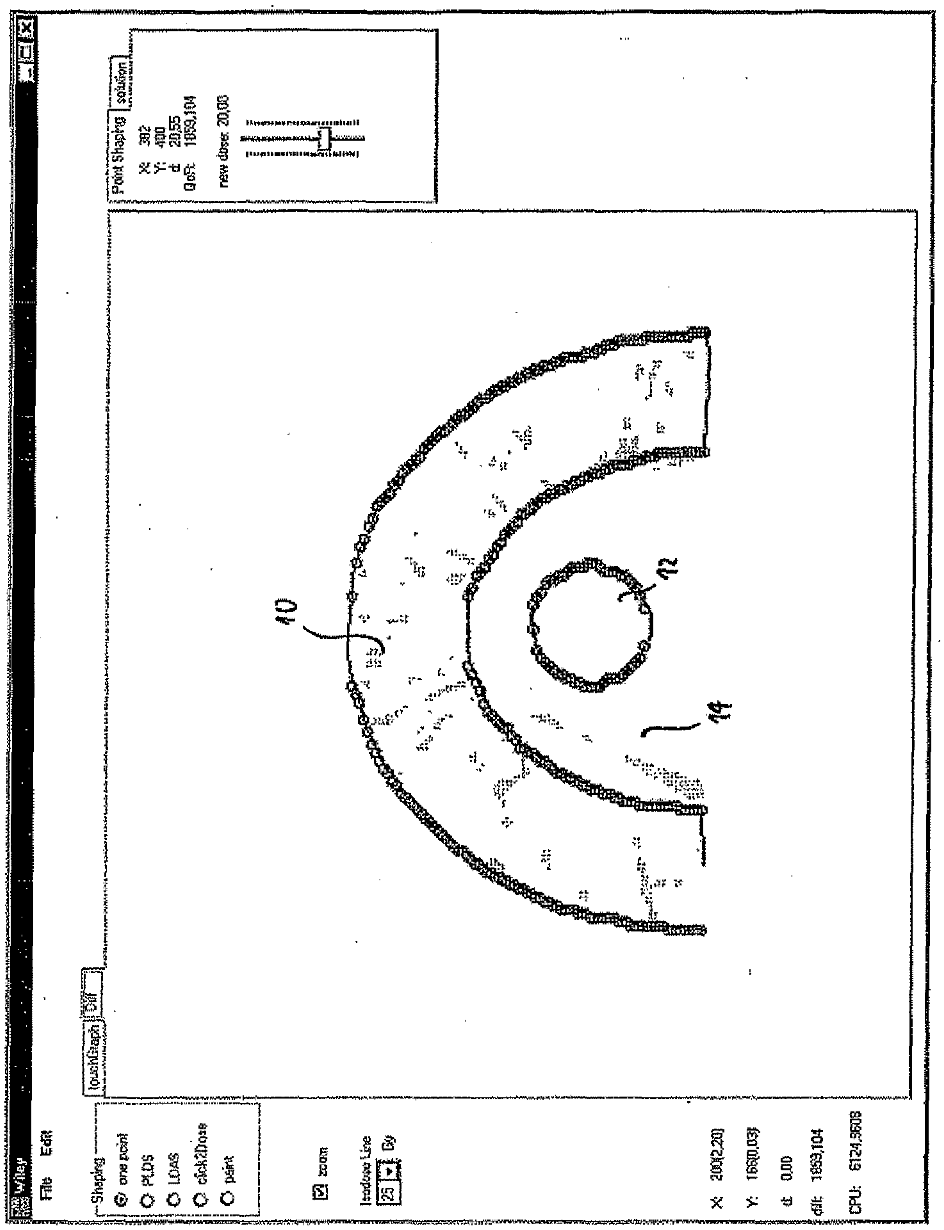
File
Edit
Shaping
one point
PLDS
LDAS
click2Dose
paint
zoom
Isodose Line
touchGraph
Diff
Point Shaping
solution
X: 392
Y: 400
d: 20,55
QoF: 1859,104
new dose: 20,00
X: 200(2,20)
Y: 169(0,03)
d: 0,00
dif: 1859,104
CPU: 6124,5608
10
12
14

Fig. 15

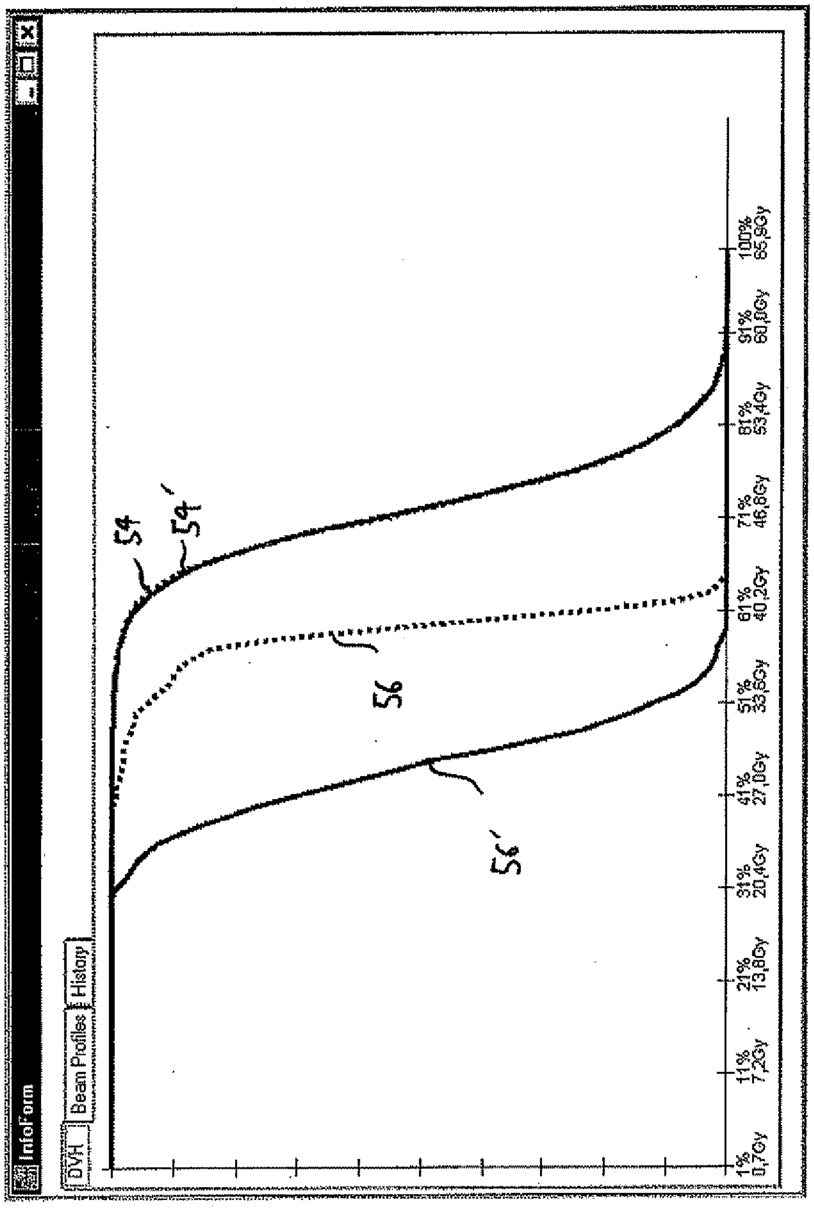
InfoForm
DVH
Beam Profiles
History
1% 0,7Gy
11% 7,2Gy
21% 13,8Gy
31% 20,4Gy
41% 27,0Gy
51% 33,6Gy
61% 40,2Gy
71% 46,8Gy
81% 53,4Gy
91% 60,0Gy
100% 65,9Gy
54
54'
56
56'

Fig. 16

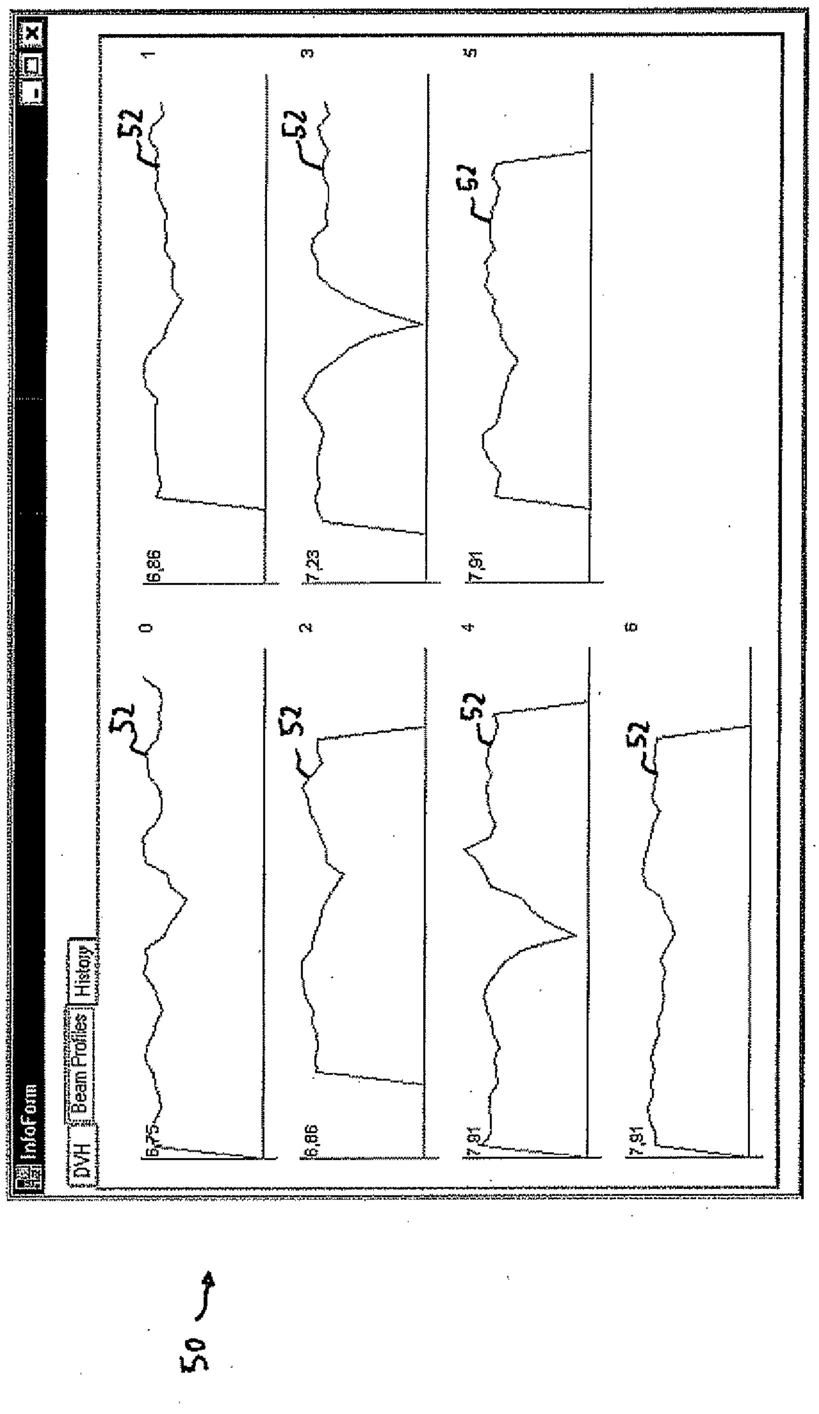
InfoForm
DVH
Beam Profiles
History
6,75
6,86
7,23
7,91
0
1
2
3
4
5
6
52
50

Fig. 17

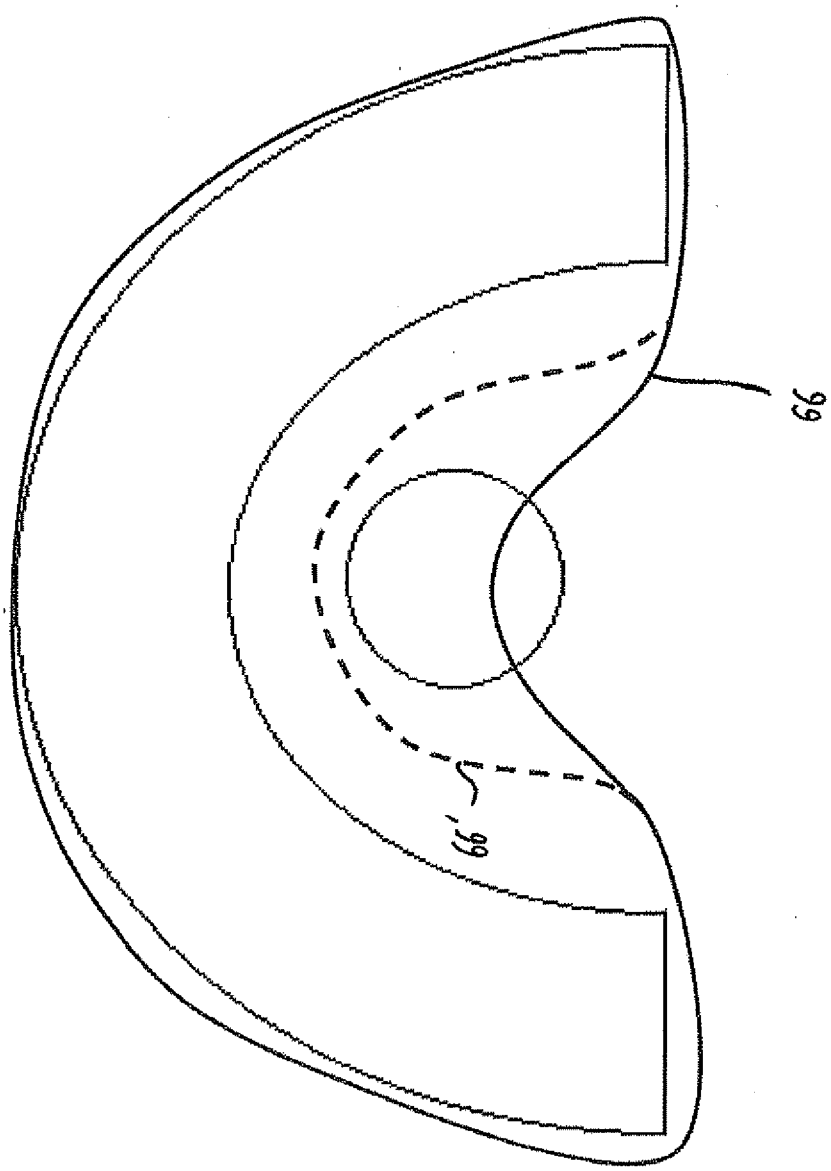


Fig. 18

Europäisches Patentamt
European Patent Office
Office européen des brevets

# EUROPEAN SEARCH REPORT

Application Number
EP 09 16 2436

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/111621 A1 (RIKER ROBERT [US] ET AL) 26 May 2005 (2005-05-26)<br>* paragraphs [0003], [0037], [0047], [0086], [0091], [0109], [0113], [0139] *<br>----- | 1-3, 13-15 | INV. A61N5/10 |
| X | COTRUTZ CRISTIAN ET AL: "IMRT dose shaping with regionally variable penalty scheme"<br>MEDICAL PHYSICS, AIP, MELVILLE, NY, US, vol. 30, no. 4, 1 April 2003 (2003-04-01), pages 544-551, XP012012031<br>ISSN: 0094-2405<br>* abstract *<br>* paragraphs [00I.], [0II.], [III.A.2.] *<br>* figures 4-6 *<br>* page 546, right-hand column *<br>* page 550, right-hand column *<br>----- | 1-7, 10-12, 14,15 | |
| X | US 2007/003011 A1 (LANE DEREK G [US] LANE DEREK GRAHAM [US])<br>4 January 2007 (2007-01-04)<br>* claim 5 *<br>* figure 1 *<br>* paragraphs [0005], [0011], [0121] *<br>----- | 1,2,4, 6-10,14, 15 | TECHNICAL FIELDS SEARCHED (IPC)<br>A61N |

The present search report has been drawn up for all claims

1

| Place of search | Date of completion of the search | Examiner |
|---|---|---|
| The Hague | 20 October 2009 | Schembri, Valentina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..............................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 09 16 2436

This annex lists the patent family members relating to the patent documents cited in the above-mentioned European search report.
The members are as contained in the European Patent Office EDP file on
The European Patent Office is in no way liable for these particulars which are merely given for the purpose of information.

20-10-2009

| Patent document cited in search report | Publication date | Patent family member(s) | Publication date |
|---|---|---|---|
| US 2005111621 A1 | 26-05-2005 | AU 2004279424 A1 | 21-04-2005 |
| | | CA 2540602 A1 | 21-04-2005 |
| | | EP 1687064 A2 | 09-08-2006 |
| | | JP 2007509644 T | 19-04-2007 |
| | | KR 20060126454 A | 07-12-2006 |
| | | WO 2005035061 A2 | 21-04-2005 |
| US 2007003011 A1 | 04-01-2007 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6661870 B

### Non-patent literature cited in the description


- **Nill, S.** Development and application of a multi-modality inverse planning system. *Ph.D. thesis, http://www.ub.uni-heidelberg.de/archiv/1802*